# EUROPÄISCHE PATENTSCHRIFT

(11) **EP 1 486 230 B1**
(45) Veröffentlichungstag und Bekanntmachung des Hinweises auf die Patenterteilung: **25.04.2007**
(21) Anmeldenummer: 03090169.8
(22) Anmeldetag: 03.06.2003
(51) Int. Cl.: A61M 37/00, A61N 5/10

(54) **Gerät und Verfahren zur Beladung von Implantationshohlnadeln mit Strahlungsquellen aus Strahlungsquellenketten zur interstitiellen Brachytherapie von Gewebe**
Device and method for loading hollow implantation needles with radiation sources from rows of seeds for interstitial brachytherapy of tissue
Appareil et procédé pour charger des aiguilles d' implantation creuses avec des sources de radiation issues de sources de radiation placées en chaîne pour la curiethérapie interstitielle de tissu

(43) Veröffentlichungstag der Anmeldung: 15.12.2004
(73) Patentinhaber: Eckert & Ziegler Eurotope GmbH, 13125 Berlin (DE)
(72) Erfinder: Löffler, Edgar, 13187 Berlin (DE); Mondry, Günther, 13125 Berlin (DE); Bornschein, Florian, 10557 Berlin (DE); Müller, Andreas, 12437 Berlin (DE)
(74) Vertreter: Hengelhaupt, Jürgen

(56) Entgegenhaltungen:
- EP-A- 0 172 288
- EP-A- 0 292 630
- EP-A- 0 466 681
- EP-A- 1 070 519
- WO-A-99/15235
- US-A- 5 851 172
- US-B1- 6 273 851
- US-B1- 6 497 645

## Beschreibung

Die Erfindung betrifft ein Gerät sowie ein Verfahren zur Konfektionierung und zur Zuführung von Strahlungsquellen aus Strahlungsquellenketten gemäß dem Oberbegriff der Ansprüche 1 und 16 sowie eine Strahlungsquellenkette gemäß dem Oberbegriff des Anspruchs 22 und einen Abstandshalter hier für gemäß dem Oberbegriff des Anspruchs 29.

Das Gerät soll die Konfektionierung und Zuführung der Strahlungsquellen vereinfachen und sicherer ausführen lassen.

Es sind bereits Verfahren und Vorrichtungen für die permanente, interstitielle Brachytherapie von Gewebe mittels Strahlungsquellen bekannt.

In der US 5,242,373 stellt Scott ein Instrument zur Implantation von radioaktiven Seeds in den Körper vor, das aus mehreren Komponenten besteht: einer statischen, dünnen Stützstange oder Stützkolben und einer weiteren dünnen Stange (Mandrin), welche durch zwei Klemmblöcke in einem konstanten Abstand gehaltenen wird. Der Mandrin ist mittels durchgehender Bohrungen in den Klemmblöcken in longitudinaler Richtung beweglich. Ein Block fungiert als Stopper, der andere als bewegliche Schienenführung für den Mandrin. Des weiteren wird ein längliches Magazin benutzt, das ebenfalls mit einem durchgehenden Bohrungskanal, in dem sich mehrere Seeds in einem bestimmten Abstand befinden, ausgestattet ist. Der Seedabstand wird durch Zwischenstücke aus bioresorbierbarem Material generiert. Das Gesamtsystem wird komplettiert durch Implantationshohlnädeln, die in das zu behandelnde Gewebe eingestochen werden.

Die Anwendung vollzieht sich schrittweise: Nach dem Einstechen der Hohlnadel in die gewünschte Geweberegion wird mittels eines Adapters das mit radioaktiven Seeds befüllte Magazin derart an die Hohlnadel angeschlossen, dass beide Durchgangskanäle übereinander passend ausgerichtet sind. In das proximale Ende des Magazins wird anschließend die Mandrinspitze eingeführt und mit dem beweglichen Führungsblock entlang der Hauptstange bis an den Stopper geschoben. Hieraus resultiert eine Zuführung des gesamten Magazininhaltes bis in die Spitze der Hohlnadel. Die zu Beginn jeder Magazinzuführung erfolgende Einstellung des Abstandes zwischen dem Stopper und dem beweglichem Führungsblock, also der Schublänge, ist somit ein kritischer Parameter für eine korrekte Seedzuführung in die Nadelspitze.

Anschließend wird das nunmehr leere Magazin entlang des Mandrins langsam bis zum Stopper zurückgezogen. Durch die Kopplung zwischen Magazin und Nadel wird auch die Hohlnadel aus dem Körper zurückgezogen und die Seeds und Abstandshalter verbleiben im Körper des Patienten.

In der US 5,860,909 wurde dieser Ansatz weiterentwickelt. Im Unterschied zur US 5,242,373 besteht dieses System aus einer Konfiguration von gegeneinander verschiebbaren Elementen innerhalb nur einer Bewegungsachse.

Der Applikator umfasst eine Hohlnadel, die in der üblichen Weise in die Prostata eingestochen wird. Durch einen Federverschluss wird die Nadel mit einem Spannfutter verbunden, welches sich verschiebbar auf einem Grundkörper des Applikators befindet. Der Grundkörper verbleibt, bezogen auf die Ausrichtung zum Patienten und für die Dauer der Implantation, stationär. Ein Seedmagazin, welches hier keine Abstandshalter, sondern ausschließlich radioaktive Strahler enthält, wird in einer vorbestimmten Ausrichtung senkrecht zur Bewegungsachse in das Spannfutter gesteckt. Die Seeds werden im Magazin durch eine Federklemme gehalten, die dafür sorgen soll, dass sich jeweils ein Seed in einem durch zwei gegenüberliegende Bohrungen gebildeten Durchgangskanal befindet. Mit Hilfe eines Mandrins, der durch den nach dem Zusammenschluss gebildeten Applikationskanal fährt, wird ein Seed durch den Hohlraum des Applikators bis zur Spitze der eingerasteten Nadel befördert.

Für die relative Bewegung des Spannfutters zum Grundkörper stehen vorgegebene Raster verschiedener Abstände zur Verfügung, um die Nadel relativ zum Applikatorkörper bei konstanter Position des Mandrins zurückzuziehen und somit ein Seed im Inneren der Prostata abzulegen. Die Nadelspitze befindet sich anschließend im vorher festgelegten Abstand zu dem ausgeworfenen Seed. Die Zuführung der gewünschten Anzahl von Strahlern in die Nadelspitze wird durch das wiederholte Zurückziehen und Vorschieben des Mandrins realisiert, wobei jeweils ein Seed durch die Federkraft im Magazin in den Einschubkanal gelangen soll.

Ein Nachteil dieser Systeme besteht in der mangelnden Zuverlässigkeit. So kann es zu einem Verklemmen der Strahler im Magazin kommen oder einem Verbiegen des Mandrins, so dass der Eingriff unter- bzw. abgebrochen oder ein neues Magazin oder Applikator verwendet werden muss. Außerdem können mehreren Seeds an dieselbe Stelle implantiert werden.

Ein weiterer Nachteil besteht in der Notwendigkeit mehrere Magazine verwenden zu müssen, da ein solches Magazin nur maximal 15 Seeds aufnehmen kann, jedoch bis zu 100 Seeds - in Abhängigkeit der Prostatagröße - für eine Operation benötigt werden.

Eine faseroptische Überwachung des Seedauswurfes ist in WO 97/22379 beschrieben. Das dortige Implantationssystem besteht aus einer integrierten Hohlnadel mit einer durchgehenden Bohrung, die eine Seedzuführung ermöglicht. Eine ebenfalls in das System integrierte dünne Stange besitzt eine Länge derart, dass im System magazinierte Seeds aus dem System durch die Nadel hindurch im Gewebe implantiert werden können. Ein kombiniertes optisches Modul bietet eine visuelle Unterstützung für den behandelnden Arzt, indem Ausführung und Ergebnis des Seedabwurfes kontrolliert werden können.

Die Bedienung erfolgt manuell mit einem Zughebel, der an einem Griff befestigt ist und die Implantation der Seeds steuert.

Die Strahler sollten ihren Implantationsort nach der Operation nicht verändern, jedoch ist eine solche Seedbewegung im Körper bekannt. Auch wenn der Verlust von einzelnen Seeds nicht unbedingt einen großen Qualitätsverlust bezüglich der verabreichten therapeutischen Dosis auf das Organ nach sich zieht, ist man trotz allem bestrebt, Vorfälle solcher Art zu unterbinden, da einzelne Seeds über die Urethra ausgeschieden werden können.

Im Gegensatz zu der Verwendung von einzelnen, losen Seeds existiert eine weitere Methode, in der die Implantate in einer fest verbundenen Konfiguration mittels der Verwendung eines bioresorbierbaren Materials (z.B. chirurgisches Nahtgut), in welches die Quellen eingebracht sind, ins Gewebe eingesetzt werden. Dadurch soll dem obigen Risiko der Seedwanderung im Körper entgegengewirkt werden.

Diese Art von Seedketten oder -schnüren werden beispielsweise aus den Strahlern selber, eventuell zusätzlichen Abstandshaltern sowie einem resorbierbaren Material, in das die Implantate eingeschlossen sind und zusammengehalten werden, gebildet.

Bereits in dem Artikel von Palos et al. (Int. J. Rad. Oncol. Biol. Phys.1980, Vol. 6, pp. 381-385) beschreibt der Autor detailliert das Einbringen von Seeds in resorbierbares Nahtmaterial zur permanenten Implantation in erkranktes Gewebe.

In einem weiteren Artikel von Scott (Surgery, Gynecology & Obstetrics, 1976, Vol. 142, pp. 667-670) werden verschiedene Implantationsmethoden für die Anwendung von Iod-125 Seeds in resorbierbarem Vicryl-Nahtgut vorgestellt, welche jedoch alle mit einem relativ hohen manuellen Aufwand verbunden sind.

In dem EP 0 292 630 präsentiert Horowitz mehrere Zuführsysteme für die Bestrahlungstherapie diverser Anwendungen, unter anderem auch für die interstitielle Brachytherapie von Prostatakarzinomen. In einer hervorgehobenen Ausführung werden die radioaktiven Seeds in einem bioresorbierbaren Material der bereits erwähnten Art in einer vorbestimmten Anordnung (z.B. axial) eingebracht. Die Trägersubstanz sollte vorzugsweise ein bis vierzehn Tage ihre Stabilität behalten und innerhalb von 70 bis 120 Tagen nach der Implantation im Gewebe absorbiert sein.

Die Quellenform kann beispielsweise als Nadel bzw. als Zylinder mit einer - bezogen auf den Durchmesser - wesentlich größeren Länge mit einer flach zulaufenden Spitze ausgeführt werden. Hierbei sind wiederum verschiedene Mechanismen bezüglich der Zusammensetzung und Konfektionierungsmethode aufgeführt. Die Bereiche zwischen den Seeds können mit entsprechenden Vertiefungen, welche als "Sollbruchstelle" fungieren, versehen sein. Eine weitere Möglichkeit besteht darin, Segmente aus vom Trägermaterial umhüllten Seeds derart zu bilden, dass mittels zueinander passenden bodenseitigen Vertiefungen und kopfseitigen Erhöhungen (mit einem etwas geringeren Durchmesser) die gewünschte Strahlerform durch Zusammenstecken entsteht. In einer anderen Ausführung werden Seeds und Abstandshalter aus bioresorbierbarem Material in einen Hohlschlauch aus obigem Trägermaterial eingeführt, dessen Spitze wiederum punktförmig ausgearbeitet ist.

Eine wesentliche Eigenschaft des Trägermaterials besteht in der Härte des resultierenden Strahlers, derart, dass er kontrolliert und in einer geradlinigen Bewegung, ohne durch Deformationen diesen Vorgang zu behindern oder zu erschweren, in das Tumorgewebe eingeführt werden kann.

Aus US 6,273,851 sind Seedketten bekannt, die aus einer radiotransparenten zylindrischen Kapsel, die ein radioaktives Isotop beinhaltet, bestehen. Die Ausführungsbeispiele der Kapseln sind dabei so gestaltet, dass eine weitgehend isotrope Strahlungsverteilung erreicht wird.

Aus US 6,273,851 sind Seedketten bekannt, die aus einer radiotransparenten zylindrischen Kapsel, die ein radioaktives Isotop beinhaltet, bestehen. Die Ausführungsbeispiele der Kapseln sind dabei so gestaltet, dass eine weitgehend isotrope Strahlungsverteilung erreicht wird. Jedoch sind die vorgestellten Seedketten schwer zu reinigen, da sie aus separaten, zueinander in Teilen beabstandeten Modulen bestehen, die erst vor Einbringen in eine Hohlnadel zusammengesetzt werden müssen.

Aus der EP 0466681 A1 ist ein Strahlerhalter zum Einsatz in interstitiell eingebrachte Bestrahlungsnadeln sowie ein Verfahren und eine Einrichtung zur Herstellung desselben bekannt. Hierbei werden Strahlungsquellen und Füllteile in einen Schrumpfschlauch eingebracht, der anschließend unter Wärmeeinwirkung geschrumpft wird. Die vorgestellte Strahlungsquellenkette weist jedoch den Nachteil auf, dass die hergestellte Kette nicht flexibel ist und somit nicht platz sparend gelagert werden kann. Langton legt in EP 0 668 088 einen Apparat und eine Methode zur Herstellung bzw. Behandlung einer Trägeranordnung für radioaktive Seeds offen. Der Seedträger besteht wiederum aus bioresorbierbarem Material, beispielsweise aus geflochtenem Vicryl mehrerer Fasern.

Der Apparat besteht aus einem stabförmigen Behältnis, welches eine durchgehende, Fläche aufweist, dadurch gekennzeichnet, dass sich darin Vertiefungen konstanten Abstandes - für die Seeds einerseits sowie (dazwischenliegend) für das Nahtmaterial andererseits - befinden, so dass die gesamte Strahlerkette eingepasst werden kann.

Eine Schutzhülle, geeignet, um die von den Seeds emittierte ionisierende Strahlung zu absorbieren, kann über die Anordnung geschoben werden.

Eine wesentliche Intention dieser Anordnung besteht darin, das vorgefertigte Trägermaterial mit den umschlossenen Seeds in der obigen Vertiefungsform zu fixieren, so dass der Abstand zwischen den Seeds konstant bleibt. Anschließend wird die gesamte Anordnung einem spezifischen thermischen Prozess unterzogen. Als Konsequenz gewinnt die umhüllte Strahlerkette eine spezifische Steife, die von Prozessparametern (Temperatur, Druck, Zeit, etc.) abhängt. Durch die vorgegebenen Abstände der Seedvertiefungen behalten die Strahler während und nach dem thermischen Prozeß ihren Abstand. Die gewonnene Festigkeit dient der verbesserten Handhabung beim Umgang der Seedkette, insbesondere während des Einführens in eine Hohlnadel.

Im zunehmenden klinischem Einsatz zeigte sich jedoch bald ein gravierender Nachteil dieser Ausführung: Dieser besteht darin, dass es hierbei zu Verstopfungen der Seedkette innerhalb der Implantationshohlnadel kommen kann, insbesondere dann, wenn Flüssigkeiten (z.B. Blut) in das Nadelinnere eindringen und mit den Strands in Berührung kommen. Eine exakte Platzierung innerhalb der Prostata wird dadurch erschwert. Es kommt außerdem zu Verzögerungen während des Eingriffes und die Menge an unbrauchbar gewordenen Strands wirkt sich negativ auf die Kosten der Operation aus.

Es besteht deshalb der Bedarf für eine Anordnung aus zusammenhängenden radioaktiven Seeds, die dahingehend optimiert wird, eine sichere Führung und einen reibungsarmen Durchgang durch Hohlnadeln zu gewährleisten.

Ein weiterer Nachteil der Ausführung nach EP 0 668 088 besteht in der Handhabung bezüglich Vorbereitung und Umgang mit den Strahlerketten nach der Auslieferung an die Anwender: Der oben beschriebene Container enthält maximal 10 Seeds. Um eine Implantation vorzubereiten, besteht die Notwendigkeit, gemäß des Operations- und Dosisplans, die benötigte Anzahl an Seeds zu konfektionieren. Dazu müssen - nachdem die äußere Hülle des Containers entfernt ist - manuell die Verbindungen zwischen den Seeds durchtrennt werden, um die nach dem Dosisplan gewünschte Anzahl von der Seedkette abzuschneiden und zu entnehmen. Hierbei kommt es zu einer Strahlenbelastung des Personals, welches diese Implantationsvorbereitungen durchführt.

Schließlich wird in der PCT Anmeldung WO 00/64538 von Reed ein weiterer radioaktiver Strahler für die Brachytherapie vorgestellt. Das verwendete Trägermaterial soll bioresorbierbar, im Wesentlichen steif und in der Form eines länglichen homogenen Stranges ausgeführt sein. Es werden des weiteren diverse Methoden zum Einbringen und Fixieren der Seeds beschrieben.

In EP 1 070 519 beschreibt Kindlein ein modulares Gerät sowie eine Methode, die Einzelseeds und Abstandshalter aus separaten kreisförmigen Magazinen prozessorgesteuert in Hohlnadeln einführt, welche bereits in der vorbestimmten Implantationsstelle im Gewebe stecken. diese nach erfolgter Seedeinführung zurückzieht, wobei der Schubdraht seine maximale Vorschubposition während des Rückzugsvorganges beibehält, bewirkt, dass die Implantationanordnung aus Seeds und Abstandshaltern im Gewebe verbleibt.

Ein Nachteil dieses Systems besteht in der Verschleppung einer eventuellen Kontamination von Körperflüssigkeiten, z.B. Blut, durch den Vorschubdraht, der durch seine Führung mit einer relativ großen Fläche im Inneren des Systems, unter anderem der Magazine von Seeds und Abstandshaltern, in Berührung kommt.

Die bekannten automatisierten Systeme haben einen relativ komplexen Aufbau, verbunden mit einem verstärkten Einsatz von elektronischen Bauteilen, an die besondere Anforderungen im klinischen Einsatz zu richten sind. Des weiteren erhöht sich die Störanfälligkeit der betreffenden Geräte, wobei eine besondere Problematik sich aus der Reinigungs- bzw. Sterilisationsnotwendigkeit ergibt, deren Ausführung und Anwendung auf solche empfindlichen Teile stark eingeschränkt ist.

Alle Systeme sind außerdem für die Zuführung von Seedketten nicht geeignet, obwohl diese einen Verwendungsanteil von ca. 50% innerhalb der interstitiellen Brachytherapie der Prostata auf dem europäischen Markt haben.

Aufgabe der Erfindung ist es, ein Gerät und ein Verfahren zur Konfektionierung von Strahlungsquellen aus Strahlungsquellenketten und eine Strahlungsquellenkette zur interstitiellen Brachytherapie von Gewebe zu entwickeln, mit denen die Nachteile der bekannten Vorrichtungen, Verfahren und Strahlungsquellenketten vermieden werden und mit denen eine einfache und sichere Handhabung bei der Zuführung von einer für eine Implantation vorherbestimmten, genau dosierten Menge von zusammenhängenden Strahlungsquellen (Seeds) in Hohlnadeln, die sich innerhalb oder außerhalb eines zu behandelnden Gewebes befinden, gewährleistet werden, wobei durch den konstruktiven Aufbau eine sichere Führung und ein reibungsarmer Durchgang der zusammenhängenden Seeds durch Hohlnadeln, eine Minimierung der Störanfälligkeit und eine optimale Reinigung bzw. Sterilisation und ein zuverlässiger Strahlenschutz gegeben sein sollen.

Diese Aufgabe wird durch die Merkmale des Gerätes gemäß Anspruch 1 und durch die Merkmale des Verfahrens zur Konfektionierung und Zuführung von Strahlungsquellenketten nach Anspruch 16 sowie durch die Merkmale der Strahlungsquellenkette nach Anspruch 22 sowie durch einen Abstands halter nach Anspruch 29 gelöst.

Das Gerät zur Beladung von Implantationshohlnadeln mit Strahlungsquellen in Strahlungsquellenketten zur interstitiellen Brachytherapie von Gewebe ist dadurch gekennzeichnet, dass mindestens ein gelagertes, antreibbares, zylinderförmiges Magazin zum Aufwickeln der Strahlungsquellenkette, mindestens ein Antriebssystem für den Transport der Strahlungsquellenkette aus dem Magazin in ein Führungssystem vorgesehen ist. Dabei wird die Strahlungsquellenkette soweit in dem Führungssystem vorgeschoben, dass sie unter einer Trennvorrichtung so zu liegen kommt, dass eine Teilkette, die eine definierte Anzahl von Seeds enthält, abgetrennt werden kann. Weiterhin enthält das Gerät die schon angesprochene Vorrichtung zum Durchtrennen der Strahlungsquellenkette, sowie eine Kontrolleinrichtung zur Überprüfung der korrekten Positionierung der Strahlungsquellenkette vor dem Durchtrennen, damit die Ummantelung der Strahlungsquellen beim Trennvorgang nicht beschädigt wird. Die abgetrennte Teilkette wird dann durch denselben oder weitere Antriebe bis in die Spitze einer angeschlossenen Hohlnadel vorgeschoben. Alle wesentlichen Bestandteile des Gerätes sind von einem strahlungsabsorbierenden Gehäuse umschlossen.

Das Verfahren zum Konfektionieren einher Strahlungsquellenteilkette und zum Einbringen dieser Teilkette in Hohlnadeln ist dadurch gekennzeichnet, dass eine Vorrichtung zur Zuführung und zur Durchtrennung von Strahlungsquellenketten aus einem Magazin an die Hohlnadel angekoppelt wird, wobei die Strahlungsquellenkette entsprechend der gewünschten Anzahl von Seeds aus dem Magazin herausgefördert, der Durchtrennvorrichtung zugeführt, dort abgetrennt und die abgetrennte Teilkette bis in die Hohlnadel eingebracht wird.

Die Strahlungsquellenkette, bestehend aus Strahlungsquellen und bioresorbierbaren Abstandshaltern im Wechsel, zusammengehalten von einer ebenfalls bioresorbierbaren Umhüllung, ist durch die kennzeichnenden Merkmale des Anspruchs 22 gekennzeichnet.

Die Geometrie der Abstandshalter ist dabei im wesentlichen zylindrisch. Der Durchmesser der Abstandshalter darf den Durchmesser der verwendeten Seeds nicht überschreiten und die Länge der Abstandshalter ist so zu wählen, dass der Abstand benachbarter Seeds in der Kette dem therapeutischen Standard entspricht. In Besonderheit weisen die hier verwendeten Abstandshalter eine Taillierung auf. Die Länge einer solchen Strahlungsquellenkette ist so groß, dass sie die Gesamtmenge an Seeds enthält, die für die Behandlung eines Patienten notwendig ist. Diese Strahlungsquellenkette wird in einem Magazin mit strahlenabschirmender Umwandung eingebracht. Dieses Magazin dient als Lagerbehälter. Es kann an das Gerät angekoppelt werden. Ein Antriebsmechanismus bewegt das Magazin und weitere Führungselemente derart, dass die Kette aus dem Magazin herausbewegt und in das Führungssystem des Geräts eingeführt wird.

Die Strahlungsquellenkette wird konfektioniert, das heißt, es werden Teilketten, welche die jeweils therapeutisch geforderte Menge an Seeds enthalten, abgetrennt und in die jeweilige Implantationshohlnadel eingeführt. Dabei kann die Nadel bereits in den Patienten eingeführt sein oder sie wird operationsvorbereitend befüllt.

Der gesamte Ablauf ist mit wenigen Eingriffen des Anwenders möglich, so dass ein bestmöglicher Strahlenschutz gewährleistet ist. Das Gerät ist so ausgelegt, dass eine zuverlässige Reinigung und Sterilisation möglich ist.

Die Anwendung des Gerätes und des Verfahrens nach der Erfindung gewährleisten, dass eine Anzahl von Seeds zur Verfügung steht, die ausreicht, um eine Implantation zum Beispiel in die Prostata durchzuführen.

Sollte am Ende der Behandlung noch ein Strahlenkettenrest übrig bleiben, so kann dieser in das Magazin zurückbewegt werden und dann mit dem Magazin entsorgt werden.

Das Magazin selbst besteht in einer bevorzugten Ausführung aus einer zylinderförmigen Speicherrolle oder Spule, auf der die Strahlungsquellenkette gelagert wird, indem sie um die Rolle gewickelt ist. Das gesamte Zuführsystem und das Magazin sind von einem Gehäuse umgeben, welches ausreichenden Schutz vor der von der Strahlenkette emittierten ionisierenden Strahlung bietet.

Vorzugsweise verfügt die Spule über eine Nut mit einer auf den Außendurchmesser der Strahlungsquellenkette optimierten Tiefe, in welche die Strahlungsquellenkette eingesetzt wird, derart, dass die Nut eine Spiralform auf der Rolle beschreibt, so dass ein konstanter Biegeradius der Kette beim Auf- und Abwickeln gewährleistet bleibt.

Gleichzeitig ist der Spulenkörper Bestandteil eines Antriebs, dadurch gekennzeichnet, dass dieser die Strahlungsquellenkette oder Teile von ihr aus der Speicherposition (aufgewickelt auf der Spule) mittels eines Führungssystems bis in die Spitze einer an das Gerät angekoppelten Hohlnadel transportiert. Hierzu steht mindestens ein weiterer zusätzlicher Antrieb zur Verfügung, der die Bewegung der Strahlungsquellenkette innerhalb der Führung erzeugt.

Ein weiterer Bestandteil des Gerätes ist eine Vorrichtung zum kontrollierten Durchtrennen der Strahlungsquellenkette, dadurch gekennzeichnet, dass ausschließlich an einer vordefinierten Stelle diese Trennung erfolgt und dabei ausgeschlossen wird, dass die Seedummantelung beschädigt wird. Hiermit wird die für die Befüllung der Hohlnadel erforderliche Anzahl an verbundenen Seeds und Abstandshaltern in einer Teilkette bereitgestellt.

In einer möglichen Ausführung besteht die Trennvorrichtung beispielsweise aus einer bewegten Metallscheibe, welche die bioresorbierbaren Abstandshalter in der Mitte durchtrennt, indem sie eine (exzentrische) Kreisbewegung ausführt. Dabei ist die Scheibe am Rand ausreichend scharf und die auf den Schneidgegenstand ausgeübte Kraft hinreichend groß, um das Material der Abstandshalter durchzuschneiden, jedoch nicht ausreichend, um die Metallummantelung der Seeds zu beschädigen.

In einer anderen Ausführung wird die Scheibe durch einen messerartigen Gegenstand ersetzt, der eine vertikale Schneidbewegung ausführt.

Eine weitere Ausführung stellt eine thermische Durchtrennvorrichtung dar, hier wird z.B. mit einem Heizdraht in einer vertikalen Bewegung die Kette an der Position eines Spacers durchtrennt. Dies hat den Vorteil, dass die Beschädigung einer Seedummantelung ausgeschlossen ist.

Die Kontrolle eines definierten Schnittpunktes kann durch verschiedene Verfahren gewährleistet werden:

Ein Verfahren besteht in der mechanischen Steuerung des Antriebes für die Strahlungsquellenkette, derart, dass der Vorschub der Strahlungsquellenkette in einer Abfolge diskreter Schritte erfolgt. Durch die Wahl einer geeigneten Schrittzahl - die Schrittweite ist durch die Gesamtlänge eines Seeds und eines Spacers bereits gegeben - und einer räumlichen Anordnung des Schneidesystems, derart, dass die Strahlungsquellenkette ausschließlich mit ihren Abstandshaltern innerhalb des Schnittbereiches zum Stehen kommt, kann erreicht werden, dass die Strahlungsquellenkette immer an einer vordefinierten Stelle durchtrennt wird. Diese Methode hat den Vorteil, dass sie keine aufwendigen elektronischen Kontrollen des Schneidesystems erfordert und bietet sich an, wenn eine optische Unterscheidung zwischen Seed und Abstandshalter erschwert ist.

Lassen sich die Positionen von Seeds und Spacern in der Kette von außen optisch unterscheiden, etwa durch die oben angeführte verschiedene Einfärbung oder Markierung der Umhüllung, ist es möglich durch einfache Sichtkontrolle oder durch den Einsatz von opto-elektronischen Sensoren die korrekte Positionierung der Strahlungsquellenkette zu überprüfen.

Weiter lassen sich auch Strahlungsdetektoren oder sonstige Sensoren, welche die Lage der Seeds messen können, für eine solche Kontrollvorrichtung einsetzen.

Diese sensor- bzw. detektorgesteuerten Ausführungen lassen sich zusätzlich mit einem Mechanismus versehen, der den Trennvorgang nur zulässt, wenn die Kette korrekt positioniert ist.

Mit einem solchen Sensor- bzw. Detektorsystem ist es zudem möglich die Zahl der Kettenelemente der abzutrennenden Teilkette zu zählen, so dass man eine vom Vortrieb unabhängige Kontrolle über die Länge der jeweiligen Teilkette, d.h. Anzahl der enthaltenden Seeds, erhält.

Weitere integrative Bestandteile des Zuführsystems bilden die Führungs- oder Transportkanäle, dadurch gekennzeichnet, dass sie eine sichere und stabile Führung während der Bewegung der Strahlungsquellenkette oder konfektionierter Teile gewährleisten. Den Abschluss auf der der Hohlnadel zugewandten Seite der Führung bildet ein Adapterstück, darauf ausgelegt, an eine Implantationshohlnadel sicher anzukoppeln und einen freien Übergang vom Führungsstück in das Lumen der Hohlnadel sicherzustellen.

Der Ausgang der Koppelstelle (und somit des gesamten Gerätes) ist so gesichert, dass nur bei einer angeschlossenen Hohlnadel, das Innere der Führungswege geöffnet wird und die konfektionierten Implantationsbestandteile in den Nadelkanal übergehen können. Ein solcher Sicherungsverschluss ist beispielsweise durch mindestens zwei gegeneinander verschiebbare Lochblenden, oder mittels federbetriebener Kugelverschlüsse realisierbar.

Ein weiterer Bestandteil des Zuführ- und Konfektioniersystems ist ein zusätzlicher Antrieb für einen Vorschubdraht, der die konfektionierte Strahlungsquellenkette - nachdem sie von dem Antriebssystem der Strahlungsquellenkette in einen Zwischenbereich transportiert wurde - weiter in die Nadelspitze vorschiebt und dort verbleibt, sodass bei einem Rückzug der Nadel die konfektionierte Teilkette im Gewebe verbleibt. Die Einführung dieses separaten Antriebselementes, z.B. eines Führungsdrahtes, in das Führungssystem erfolgt über einen definierten, isolierten Zugang, so dass dadurch eine räumliche Trennung zwischen der unkonfektionierten Strahlungsquellenkette und dem Führungsdraht erfolgt, die dazu beiträgt, einer Kontaminationsverschleppung, hervorgerufen durch Körperflüssigkeiten, z.B. Blut, entgegenzuwirken.

Durch einen Steuerungsmechanismus ist sichergestellt, dass der Draht nur hinter einem Strahlungsquellenkettenstück in die Zuführung zur Nadel gelangt und nicht in das Verbindungsstück eindringen kann, wenn dieses durch die Strahlungsquellenkette blockiert ist, oder seinerseits den Vorschub der Strahlungsquellenkette behindert, so dass eine Beschädigung der Strahlungsquellenkettenumhüllung oder ihrer Bestandteile ausgeschlossen ist.

In einer anderen Ausführung - insbesondere im Zusammenhang mit der beschriebenen Methode einer Befüllung von Hohlnadeln bevor diese in das Gewebe eingestochen werden - wird auf die Verwendung eines Vorschubdrahtes verzichtet.

Des weiteren ermöglicht das Zuführsystem dem Anwender eine Vorwahl über die Anzahl der Seeds in der zu konfektionierenden Teilkette zu treffen, mit der jeweils die angekoppelte Implantationshohlnadel beladen wird. Die Umsetzung wird mittels der Steuerung des Strahlungsquellenkettenantriebs und des Schneidesystems realisiert.

In einer möglichen Ausführung erfolgt die Bereitstellung gemäß der Vorwahl über eine definierte Umdrehung aller Antriebsräder bei der Abwickelung des Strahlungsquellenkettenmagazins bis zum Schneidesystem, dadurch gekennzeichnet, dass der vorgewählten Seedmenge eine diskrete Anzahl ganzzahliger Vielfacher einer von der mechanischen Ausführung abhängigen Umdrehung entspricht. Nach dem Schneiden ist die Vorschublänge des Vorschubdrahtes auf die sich ergebende Länge des abgeschnittenen Strahlungsquellenkettenteilstückes abgestimmt, derart, dass der geometrische Endpunkt des Fahrweges der Drahtspitze am der Hohlnadel abgewandten Seite des jeweiligen Strahlungsquellenkettenteilstückes, bezogen auf seine Endstellung innerhalb der Hohlnadel, liegt. Somit besteht eine Relation zwischen der vom Anwender vorgewählten Seedanzahl und dem Vorschubweg, die als Information vom Apparat verarbeitet wird.

Das neuartige Zuführ- und Konfektioniersystem ist so modular aufgebaut, dass Bauteile, die besonders von der besagten Bio-Kontamination gefährdet sind, leicht entfernt oder gereinigt und sterilisiert werden können.

Das gesamte Gerät ist ferner so konzipiert, dass es eine Umschließung besitzt, die für eine strahlenschutzgemäße Abschirmung der durch die radioaktiven Seeds entstehenden, ionisierenden Strahlung sorgt. Insbesondere das Magazin, in dem die Strahlungsquellenkette lagert, ist so geschützt, dass es ein Einsetzen bzw. Auswechseln mit einer für eine gesamte Operation vorgesehenen Seedanzahl erlaubt und dabei die von ihm ausgehende Dosisbelastung für den Anwender im Vergleich die nach jetzigen Stand der Technik in der interstitiellen Brachytherapie üblichen Dosisbelastungen signifikant unterschreitet.

Zweckmäßige weitere Ausführungsformen der Erfindung sind in den Unteransprüchen beschrieben.

Die Erfindung wird nachfolgend in einem Ausführungsbeispiel der Erfindung für die Behandlung von Prostatakarzinom näher erläutert. In der zugehörigen Zeichnung zeigen:
- Fig. 1:: eine räumliche Übersichtszeichnung einer verwendeten Strahlungsquellenkette,
- Fig. 2a:: eine Seitenansicht der Strahlungsquellenkette nach Fig. 1,
- Fig. 2b:: eine Ausschnittsvergrößerung der Kette aus Fig. 2a, die einen Abstandshalter zeigt,
- Fig. 3:: eine Ausführung der Seed- und Abstandshalterenden,
- Fig. 4:: eine stark vereinfachte, schematische Übersicht einer Ausführung des Zuführsystems,
- Fig. 5a:: die räumliche Darstellung der Spule mit aufgewickelter Strahlungsquellenkette,
- Fig. 5b:: die Darstellung der Spule mit aufgewickelter Strahlungsquellenkette nach Fig. 5a in einer Seitenansicht,
- Fig. 6:: die schematische Darstellung des Antriebssystems zur Zuführung der Strahlungsquellenkette zum Schneidesystem,
- Fig. 7:: die schematische Darstellung des Schneidesystem,
- Fig. 8:: die schematische Darstellung des Magazins der Drahtführung,
- Fig. 9:: die schematische Darstellung der Anschlussstelle, in die der Führungsdraht in den Kanal der Strahlungsquellenkette eintritt und
- Fig. 10:: die schematische Darstellung des Endstückes des Führungssystems mit seiner Koppelstelle für die Implantationshohlnadel.

Der Prostatatumor ist mittlerweile zur häufigsten Krebserscheinung in der männlichen Bevölkerung und gleichzeitig Ursache für den zweithäufigsten Anteil an Todesfällen aufgrund von Krebserkrankungen geworden.

Neben Wärme- bzw. Kälteanwendungen und Hormonbehandlungen bietet sich vor allem die Strahlentherapie - entweder allein oder in Kombination mit anderen Verfahren - an. Ionisierende Strahlen verursachen Schäden im Erbgut der Zellen. Krebszellen haben ein weniger gut funktionierendes Reparatursystem als normale Zellen; deshalb können die verursachten Schäden schlechter behoben werden und die Krebszellen sterben ab.

Bei der temporären Brachytherapie mit der sog. Afterloading Technik werden Strahlenquellen in Abhängigkeit der zu verabreichenden Dosis nur für eine bestimmte Zeit in das Tumorgewebe eingeführt. Dies geschieht mit entsprechend plazierten Kanälen, z.B. Kathetern; die Bewegung der Quellen kann manuell oder automatisiert erfolgen.

Hierbei implantiert man kleine, gekapselte Strahlenquellen, die Radioisotope mit relativ kurzer Halbwertszeit und niederenergetischer Strahlungscharakteristik enthalten (z.B. Iod-125 oder Pd-103), in die Nähe oder direkt in das erkrankte Gewebe, wo diese dauerhaft verbleiben und dabei eine therapeutische Strahlendosis an ihre Umgebung abgeben und somit die Krebszellen zerstören. Die auftretende Dosisleistung ist im Vergleich zur temporären Therapie daher deutlich reduziert.

Ein entscheidender Vorteil der permanenten Implantation gegenüber der fraktionierten Therapieform bei temporären Implantaten ist der einmalige Eingriff am Patienten. Zudem ist aufgrund der Nuklidcharakteristik bei permanenten Implantaten der Wirkungsradius begrenzt und das umliegende gesunde Gewebe wird nicht belastet. Man ist dabei bestrebt, eine homogene Verteilung der Dosis auf das erkrankte Gewebe anzuwenden und Problemzonen, wie den Harnleiter, die Samenbläschen oder das Rektum vor zu hoher Belastung zu schützen.
Dies macht eine exakte Plazierung der Seeds in der Prostata notwendig.

Mit Hilfe von einzelnen Schichtbildern des Organs und einer Kennzeichnung des Zielgebietes durch den Arzt wird durch den Einsatz einer speziellen Software ein Plan für die Positionierung der Strahler in Abhängigkeit vom verwendeten Nuklid und Aktivität entwickelt.

Aus der Bilderfassung resultiert die Ausrichtung eines rasterartigen Koordinatensystems, welches für alle Ultraschallbilder der Prostata als Referenz dient. Dieses virtuelle Raster wird mittels einer Loch-Rasterplatte realisiert, die bezüglich der longitudinalen Ultraschallachse und deren Bewegungsrichtung ausgerichtet ist. Die Lochplatte fungiert nun während der Operation zur Orientierung für das Einstechen der Hohlnadeln.

Die Brachytherapie der Prostata erfolgt auf unterschiedliche Weisen, die sich im wesentlichen durch den Zeitpunkt der Dosisplanung und der Befüllung der Implantationsnadeln unterscheiden.

Bei der pre-operativen Dosisplanung werden die Nadeln operationsvorbereitend nach den Vorgaben des Dosisplans beladen. Dabei werden die Nadeln jeweils an das Gerät angekoppelt, die Strahlungsquellenkette entsprechend der geforderten Anzahl von Seeds für die jeweilige Nadel aus dem Magazin herausgefördert, die Teilkette wird abgetrennt und dann bis in die Spitze der Hohlnadel vorgeschoben.

Diese Nadeln werden dann im Operationssaal vom Arzt in den Patienten von Hand eingestochen und die darin befindlichen Seed-Teilketten in der Prostata mit Hilfe eines Mandrins abgelegt.

Bei der intra-operativen Dosisplanung werden zunächst die Nadeln in die Prostata unter transrektaler Ultraschall. bildkontrolle eingestochen und erst dann die Dosisplanung durchgeführt. Hier werden also nicht nur Teilketten mit der therapeutischen geforderten Anzahl von Seeds konfektioniert und in Nadeln eingebracht, sondern die Ablage der Teilketten wird direkt mit dem Gerät ausgeführt. Dies bedarf einer modifizierten Ausführung des Gerätes.

Zunächst ist der Vorgang identisch mit dem Ablauf bei der Methode der pre-operativen Planung, abgesehen davon, dass das ganze Prozedere im Operationssaal stattfindet und die anzukoppelnde Nadel sich bereits im Patienten befindet. Das Gerät wird hier über eine geeignete Halterung mit dem Zielsystemträger verbunden, damit keine ungewollten Kräfte auf die Nadel übertragen werden und der Patient nicht verletzt werden kann. Nachdem die konfektionierte Teilkette ebenfalls bis in die Spitze vorgeschoben ist und dabei von dem mandrinähnlichen Vorschubsystem dieser Gerätevariante fixiert ist, wird der Arzt die distale Ablageposition der Teilkette durch entsprechende Positionierung der Eindringtiefe der Nadel gemäß seiner Planung einstellen. Die Ablage erfolgt nun, indem ein Mechanismus des Geräts die Nadel zurückzieht, während die Teilkette von dem oben angesprochenen Vorschub in Position gehalten wird, sich also nicht mit der Nadel zurückziehen kann, sondern vielmehr in der Prostata abgelegt wird.

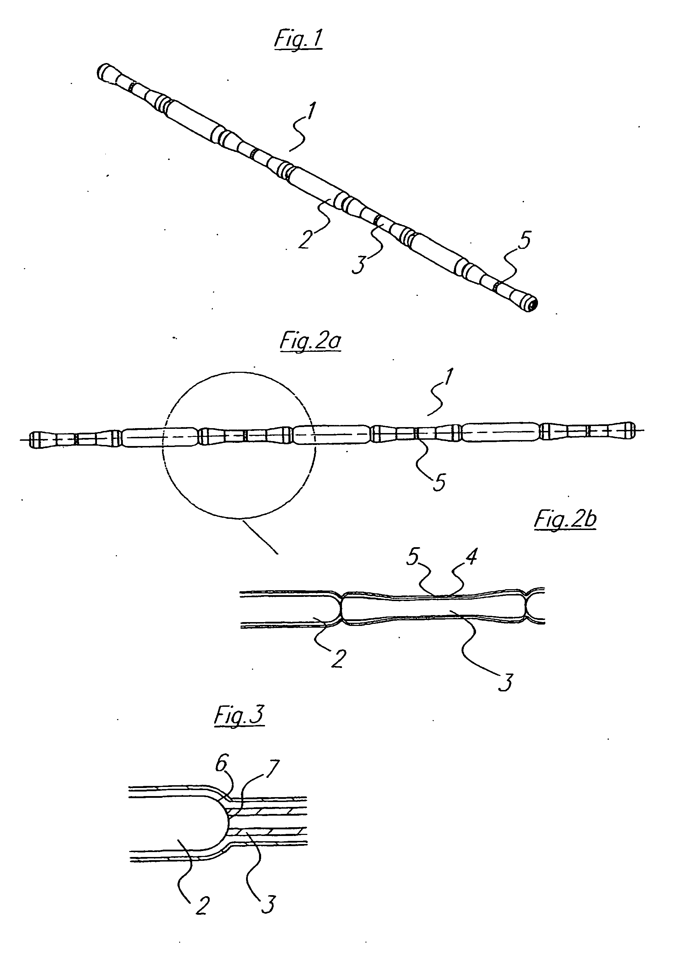

In Fig. 1 wird eine Ausführung einer verwendeten Strahlungsquellenkette 1 in räumlicher Darstellung gezeigt. Man erkennt die abwechselnd angeordneten Seeds 2 und Abstandshalter (Spacer) 3.

Fig. 2a zeigt die Strahlungsquellenkette 1 nach Fig. 1 in einer Seitenansicht und Fig. 2b die entsprechend gekennzeichnete Ausschnittsvergrößerung aus Fig. 2a als Querschnitt.

In dieser Konfiguration der Strahlungsquellenkette 1 befinden sich zwischen zwei Seeds 2 bioresorbierbare Abstandshalter 3, welche von einer Umhüllung 4, die ebenfalls bioresorbierbar ist, umgeben sind. Zur verbesserten Erkennbarkeit durch Sensoren und einem günstigeren Verhalten während des Abbaus im Körper des Patienten nach der Implantation sind die Spacer 3 tailliert ausgeführt.

Auf der Umhüllung der Strahlenquellenkette 1 sind Markierungen 5 angedeutet, welche die Position der Spacer 3 innerhalb der Kette 1 sicher identifizierbar machen sollen.

In einer möglichen Ausführungsform der Erfindung wird das von der Firma Bebig Isotopen- und Medizintechnik GmbH produzierte und vertriebene "IsoSeed^{™}" eingesetzt, und es werden Spacer 3 mit Hohlraum verwendet, siehe auch Fig. 3. Dieser Hohlraum soll die Sichtbarkeit der Spacer 3 in Ultraschallaufnahmen verbessern.

Grundsätzlich gilt, dass der Außendurchmesser der Spacer 3 kleiner oder gleich dem Außendurchmesser der Seeds 2 ist.

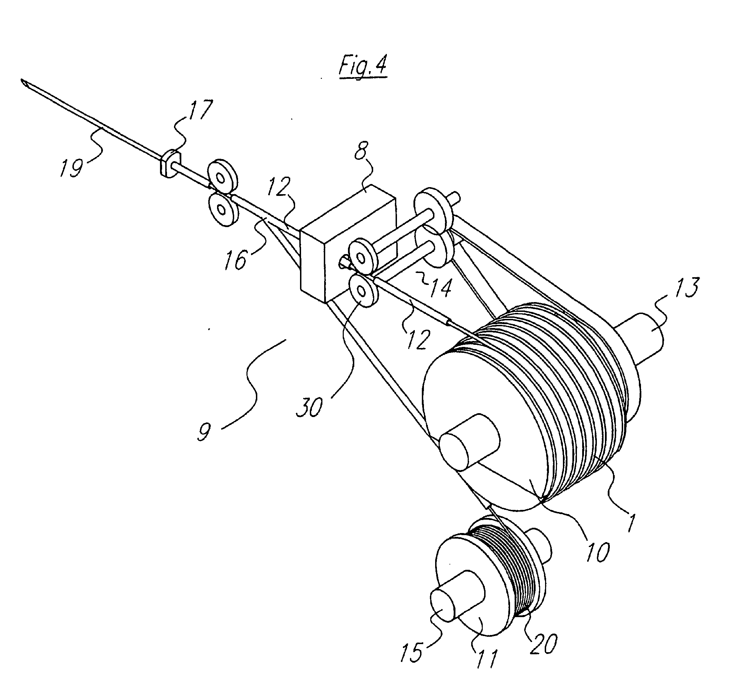

Fig. 3 illustriert eine Ausführungsmöglichkeit der Stirnenden 7 der Abstandshalter 3, hier mit Hohlraum, die unmittelbar in Kontakt zu den Stirnenden der Seeds 6 stehen und eine Stabilisierung der Bestandteile der Strahlungsquellenkette 1 realisieren. Unter Berücksichtigung der während der Lagerung auf der Spule 10 und der während der Bewegung im Führungssystem (Fig. 4) auftretenden Biege-, Stauchungs- und Zugkräfte sowie der Tatsache, dass trotz dieser Kräfte eine Konstanz bezüglich der gesamten Strahlungsquellenkettenlänge eine wichtige Vorraussetzung für eine sichere Funktion der Apparatur und der Implantationsprozedur ist, bietet eine zu den konkaven Seedenden 6 konträre konvexe Form der Abstandshalterenden 7 eine diesbezüglich optimierte Ausführung, die einerseits eine Drehung - ähnlich einem Kugelgelenk - zulässt und gleichzeitig über eine hinreichend große Kontaktfläche zur Druckübertragung verfügt.

Eine schematische Übersichtszeichnung einer möglichen Ausführung des Zuführ- und Konfektioniersystems 9 für die Strahlungsquellenkette 1 ist in Fig. 4 dargestellt.

Dabei ist nicht dargestellt, dass das Gerät von einem Gehäuse umgeben ist, welches die Strahlung nach außen abschirmt.

Als Magazin für die Strahlungsquellenkette 1 fungiert eine Spule 10 mit einer Antriebsachse 13, auf der sich die aufgerollte Strahlungsquellenkette 1 befindet. In direktem Anschluss ist der Beginn des Führungssystems positioniert, in das die Strahlungsquellenkette 1 unmittelbar nach dem Verlassen des Spulenkörpers 10 eingeführt wird. Das Führungssystem besteht aus einem Rohr 12 mit einem geeigneten Durchmesser. Zur Stabilisierung der Bewegung der Strahlungsquellenkette 1 im Führungssystem befindet sich in einem geeigneten Abstand ein zweiter Antrieb 14, bestehend aus zwei ineinanderlaufenden Rädern 30, die den von der Spule 10 abgewickelten Teil der Strahlungsquellenkette 1 aufnehmen und in Richtung eines Schneidesystems 8 transportieren.

Die Steuerung bzw. die Kopplung aller Antriebe 13, 14, die eine zusammenhängende Strahlungsquellenkette 1 bewegen, ist dadurch gekennzeichnet, dass ein synchroner Transport gewährleistet ist, der verhindert, dass Spannungen oder Stauchungen innerhalb der Strahlungsquellenkette 1 auftreten und diese beschädigen.

Durch ein weiteres Führungsrohr 12 erfolgt die Zuführung in das Schneidesystem 8, in der die vom Anwender vorgegebene Anzahl von Seeds 2 und Abstandshaltern 3 konfektioniert wird. Hieran schließt sich ein erneutes Führungsrohr 12 an, in dem die Strahlungsquellenkette 1 bis zu einem als Y-Weiche ausgeführten Anschlussstück 16 geführt wird.

Dieses als Zuführungsstück fungierende Anschlussstück 16 welches vorzugsweise ebenfalls ein aus Metall gefertigtes Rohrstück ist, dient ausschließlich als Wegstück für einen aus einem spulenförmigen Magazin 11 abgerollten Führungs- bzw. Vorschubdraht 20 , der hierüber in das Hauptzuführungsrohr 12 gelangt, um die konfektionierten Implantationsbestandteile in Richtung der Implantationshohlnadel 19 vorzuschieben. Zu diesem Zweck besitzt die Spule 11 des Führungsdrahtes 20 einen eigenen Antrieb 15.

Den Abschluss des Endstückes des Führungsrohres 12 bildet ein Kopplungsmechanismus in Form eines Adapterstückes 17, an welches sich geeignete Implantationshohlnadeln 19 anschließen lassen, derart, dass die Strahlungsquellenkette 1 ungehindert in das Nadellumen eingeführt werden kann.

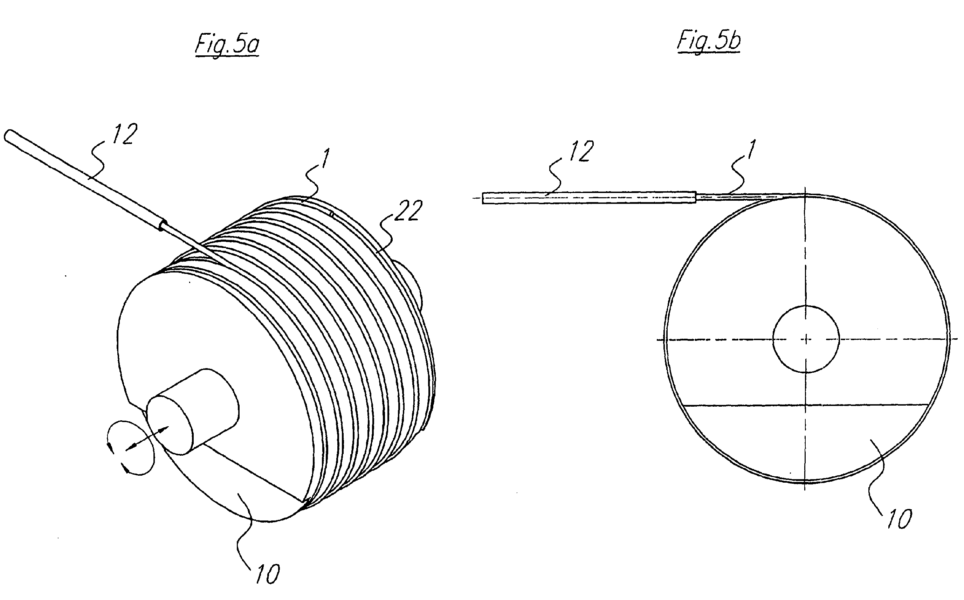

Fig. 5a zeigt die Spule 10 der Strahlungsquellenkette 1 in einer räumlichen Darstellung. In der vorliegenden Ausführung ist die Strahlungsquellenkette 1 fast vollständig auf dem Spulenkörper 10 aufgerollt. Die Strahlungsquellenkette 1 ist in einer in die Spulenkörperoberfläche eingebrachten Nut 22 eingebracht, die auf den Durchmesser der Strahlungsquellenkette 1 optimal angepasst ist und eine Stabilisierung bezüglich quer zur Richtung der Strahlungsquellenkette 1 wirkenden Kräfte bietet. Des weiteren ist die Wicklung der Strahlungsquellenkette 1 dadurch gekennzeichnet, dass ein konstanter Krümmungsradius gewährleistet ist, derart, dass die einzelnen Wicklungen spiralförmig um den Spulenkörper 10 führen und in einem definierten Abstand zueinander liegen.

In der Seitenansicht Fig. 5b ist der Übergang der Strahlungsquellenkette 1 in das Führungsrohrsystem 12 dargestellt. Aufgrund der örtlich festen Position der Rohröffnung ist die Spule 10 so gelagert, dass der Spulenkörper 10 während seiner Drehbewegung eine Translation in Richtung seiner Drehachse ausführt und somit die abgerollte Strahlungsquellenkette 1 immer senkrecht bezüglich der Spulenachse in den Rohreingang eingeführt wird.

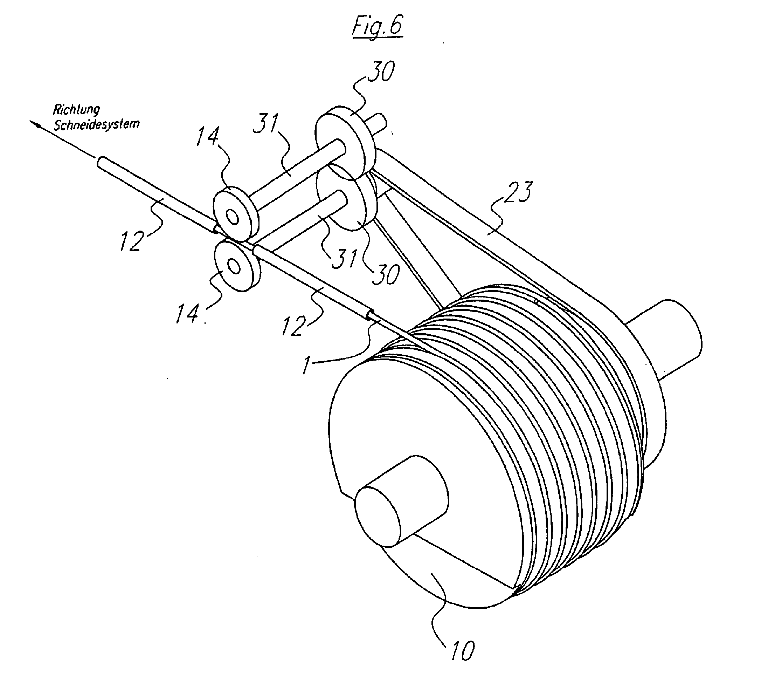

In Fig. 6 ist das zusammenhängende Antriebssystem der Spule 10 und der Sekundärantriebe in einer möglichen Ausführung dargestellt, welche die Strahlungsquellenkette 1 dem Schneidesystem 8 zuführen. Die Spule 10 ist mit einem Antriebsrad ausgestattet, das mittels einer Riemenverbindung 23 in Verbindung mit dem Sekundärantrieb 14 steht. Dieser besteht aus ineinandergreifenden Zahnrädern 30, welche über eine Welle 31 mit den eigentlichen Antriebsrädern 14 in Verbindung stehen, welche die Rotationsbewegung in eine Translation der Strahlungsquellenkette 1 umwandeln. Hierfür ist das Führungsrohr 12 unterbrochen und ermöglicht somit den direkten Kontakt mit der Strahlungsquellenkette 1. Der Anpressdruck auf die Strahlungsquellenkette 1 ist im Zusammenhang mit einem geeigneten Profil so eingestellt, dass einerseits ein Schlupf verhindert, andererseits eine Beschädigung der Strahlerbestandteile ausgeschlossen wird. Die Bewegung der Strahlungsquellenkette 1 kann sowohl in Richtung des Schneidesystems 8, als auch in umgekehrter Richtung zur Spule 10 erfolgen.

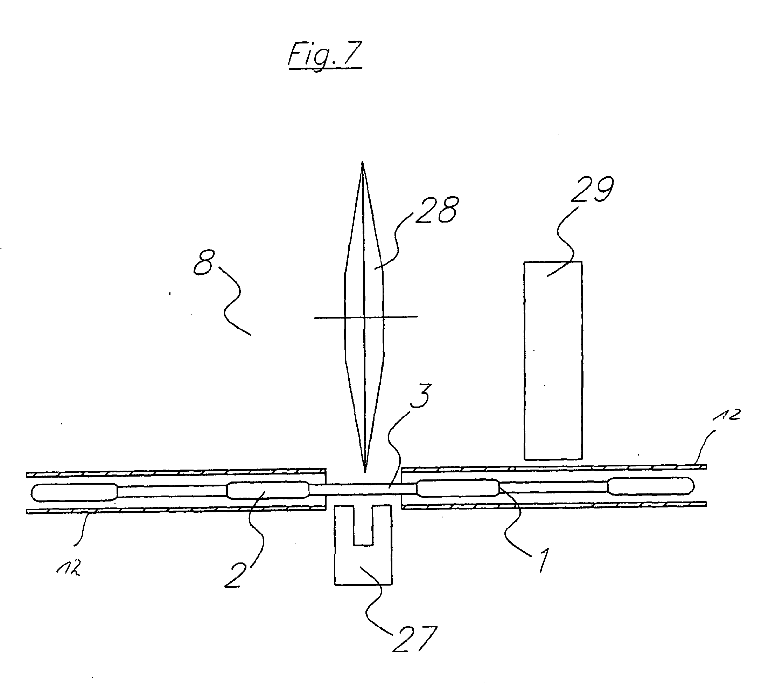

Fig. 7 zeigt einen Querschnitt des Durchtrenn- bzw. Schneidesystems 8 mit dem zur Spule 10 und zur Hohlnadel 19 führenden Führungsrohr 12, in dem sich die Strahlungsquellenkette 1 befindet. Eine gemäß der Anwendervorgabe definierte Anzahl von Seeds 2 mit dazwischenliegenden Abstandshaltern 3 liegt auf der zur Hohlnadel 19 führenden Seite der Apparatur. Die Strahlungsquellenkette 1 wird für das Durchtrennen unterhalb eines Schneidrades 28 positioniert, derart, dass sichergestellt ist, dass der Abstandshalter 3 in der Mitte durchtrennt wird. Für die Schneidbewegung führt das Schneidrad 28 eine (ggf. exzentrische) Rundbewegung aus, deren Antrieb hinreichend stark ist, um das Material der Abstandshalter 3 in einer einzigen Bewegung zu durchtrennen, wobei ein Auflagestück 27 unterhalb des Abstandshalters 3 verhindert, dass die Strahlungsquellenkette 1 durch die senkrecht zum Führungsrohr 12 wirkende Kraft aus diesem hinausgedrückt wird.

Vor dem Schneiden wird die korrekte Lage der Strahlungsquellenkette 1 bzw. des zu durchtrennenden Abstandshalters 3 mittels eines Sensors 29 kontrolliert. Am Ende des Schnittvorganges befindet sich das Schneidrad 28 wieder in seiner Ausgangsposition, so dass eine Vorschubbewegung des abgeschnittenen Teils der Strahlungsquellenkette 1 (Teilkette) ermöglicht wird.

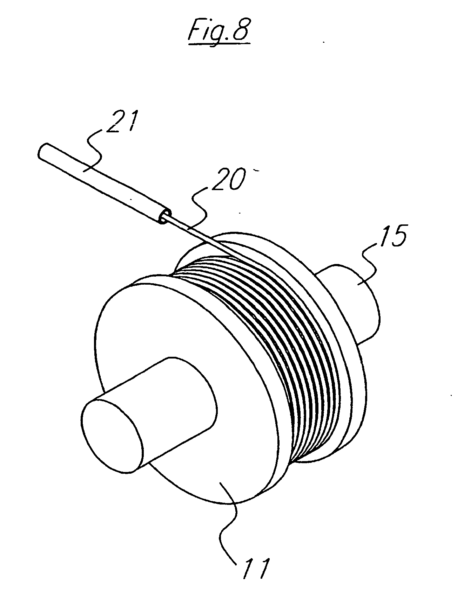

Fig. 8 zeigt das Drahtführungsmagazin 11, das hier als Spule realisiert ist. Der Draht 20 besteht aus einer Nickel-Titan Legierung und ist wie die Strahlungsquellenkette 1 mit einem konstanten Radius auf der Spule 11 aufgewickelt. Die zugehörige Antriebssteuerung ist dadurch gekennzeichnet, dass ein Vorschub des Drahtes 20 in den Hauptkanal 12, in dem sich die Strahlungsquellenkette 1 befindet, nur ermöglicht wird, wenn die Eintrittsstelle nicht blockiert ist.

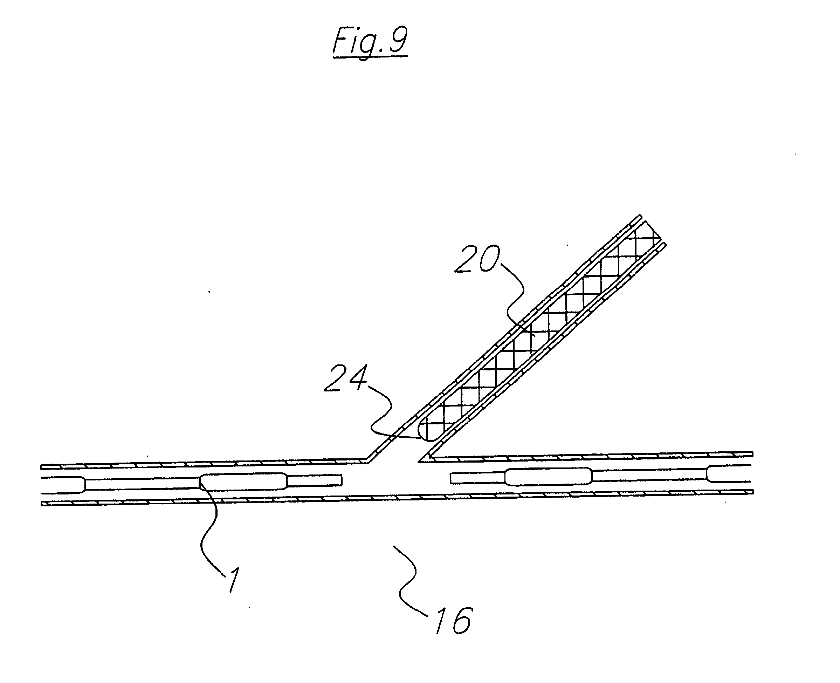

Fig. 9 zeigt die Anschlussstelle 16, hier als Y-Weiche ausgeführt, wobei der konfektionierte Teil der Strahlungsquellenkette 1 (Teilkette) bereits in Richtung der Hohlnadel 19 hinter die Eintrittsöffnung transportiert wurde, so dass der aus der Austrittsöffnung angetriebene Führungsdraht 20 hinter den abgetrennten Teil der Strahlungsquellenkette 1 (Teilkette) gelenkt werden kann und dessen weiteren Vorschub bewirkt. Die Drahtspitze 24 des Führungsdrahtes 20 ist hier, als Beispiel einer möglichen Ausführung, als Halbkugel ausgeführt und so ausgearbeitet, dass diese ein dem Führungsrohrlumen und dem Strahlungsquellenkettendurchmesser angepassten Querschnitt hat, so dass einerseits eine optimale Fläche für die auf die Strahlungsquellenkette 1 übertragene Schubkraft entsteht, gleichzeitig jedoch die Beschädigungsgefahr der Strahlungsquellenkette 1, z.B. durch scharfe Kanten, verhindert wird.

In einer anderen, nicht dargestellten Ausführung besitzt die Drahtspitze 24 einen flachen kreisförmigen Abschluss mit abgerundeten Kanten.

Die vorschublänge des Führungsdrahtes 20 ist auf die Länge der konfektionierten Strahlungsquellenkette 1 (Teilkette) in Abhängigkeit der vorgewählten Seedanzahl abgestimmt, derart, dass sichergestellt ist, dass jede konfektionierte Strahlungsquellenkette 1 bis in die Spitze der Hohlnadel 19 geschoben wird. Der mit der Spule 10 verbundene Teil der Strahlungsquellenkette 1 wird in Richtung der Spule 10 hinter das Schneidesystem 8 zurückgezogen, um anschließend die Schneideprozedur erneut zu durchlaufen.

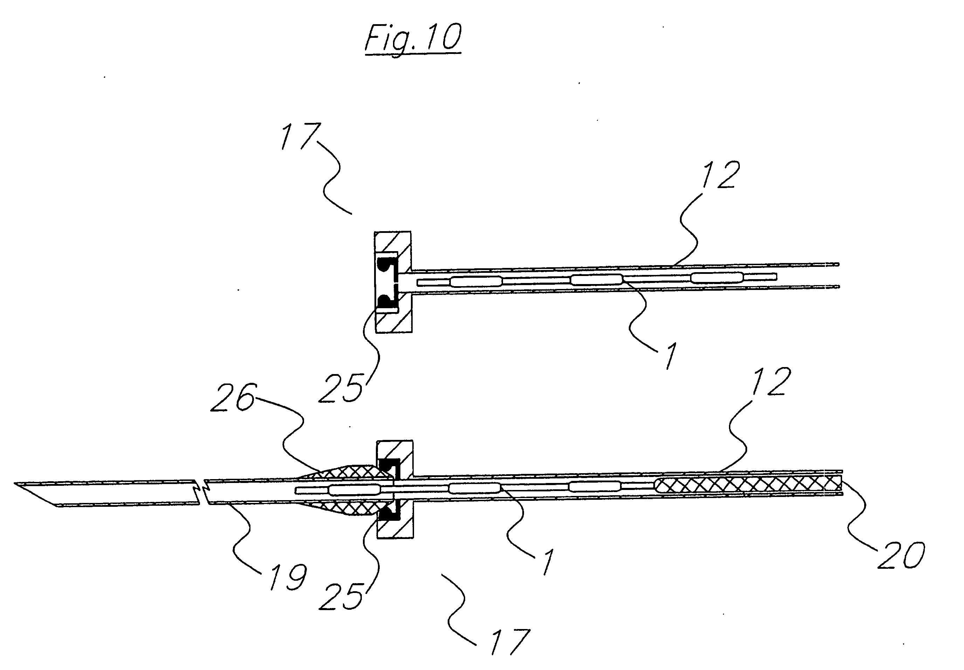

Fig. 10 zeigt die Kopplungsstelle des Gerätes mit der Hohlnadel 19 zum Konfektionieren und Zuführen mit dem Verschlusssystem im ungeöffneten (ungekoppelten) sowie im geöffneten (gekoppelten) Zustand.

Den Abschluss des Führungsrohres 12 bildet das Adapterstück 17, hinreichend groß, um das Anschlussende der Hohlnadel 19 aufzunehmen. Eine mit einem Federmechanismus ausgestattete zweiteilige Lochblende 25 verschließt den Durchgangskanal, indem die exzentrisch sitzende kreisförmige Bohrung der äußeren Blende 25 gegenüber der Bohrung der innen liegenden Blende 25 verschoben ist. Damit wird verhindert, dass unbeabsichtigt Implantationsbestandteile aus dem Gerät treten können. Zur Öffnung des Verschlusses (Adapter) 17 muss eine Hohlnadel 19 mit einem Gegenstück 26, z.B. einem weiblichen Luer-Lock Adapter, mittels einer Drehbewegung in die äußere Öffnung des Führungsrohres 12 angeschlossen werden, derart, dass sich die Bohrung der äußeren Lochblende 25 exakt über die Bohrung der inneren Blende 25 schiebt und damit ein Durchgangskanal für die Strahlungsquellenkette 1 freigegeben wird.

In einer nicht dargestellten Ausführung kann der Sicherungsverschluss des Adapterstückes 17 auch durch zwei Kugeln realisiert werden, die mittels Federkraft gegeneinander gedrückt werden. Beim Durchgang eines Nadelendstückes mit einem gegenüber dem Nadelaußendurchmesser vergrößertem Abschlussring werden die beiden Kugeln senkrecht zur Nadelbewegungsrichtung über den Ring gedrückt, so dass sie in ihrer Endposition auf der Seite hinter dem Abschlussring in einer vertieften Position liegen und somit die gesamte Nadel fixieren.

Es sei darauf hingewiesen, dass sich die Beschreibung der Erfindung hauptsächlich auf die Behandlung von Prostatakarzinomen bezieht. Dies soll jedoch keine patentschutzrelevante Einschränkung bezüglich weiterer medizinischer Anwendungen darstellen. Vielmehr sind eine Vielzahl von Brachytherapien denkbar, in denen radioaktive Strahlungsquellenketten in lebendes Gewebe zum Zwecke der Abgabe einer Energiedosis implantiert werden.

### Bezugszeichenliste

- 1: Strahlungsquellenkette
- 2: Strahlungsquelle (Seed)
- 3: Abstandshalter
- 4: Umhüllung
- 5: Markierung der Spacerposition
- 6: Seedende
- 7: Abstandshalterende
- 8: Durchtrennvorrichtung
- 9: Gerät zur Konfektionierung und Zuführung
- 10: Spulenkörper (Magazin)
- 11: Drahtmagazin
- 12: Führungsrohr
- 13: Antriebsachse
- 14: Antrieb für Strahlungsquellenkette
- 15: Antrieb für Drahtmagazin
- 16: Anschluss (Y-Weiche)
- 17: Adapterstück
- 18: Weiterer Antrieb für 1
- 19: Implantationshohlnadel
- 20: Führungsdraht
- 21: Drahtführungsrohr
- 22: Nut
- 23: Antriebsriemen
- 24: Drahtspitze
- 25: Blende
- 26: Gegenstück
- 27: Auflagestück
- 28: Schneidrad
- 29: Sensor
- 30: Rad
- 31: Welle

## Patentansprüche

1. Gerät zur Beladung von Implantationshohlnadeln mit Strahlungsquellen aus Strahlungsquellenketten zur interstitiellen Brachytherapie von Gewebe, **dadurch**
**gekennzeichnet, dass**
- mindestens ein gelagertes, antreibbares, zylinderförmiges Magazin (10) zum Aufwickeln der Strahlungsquellenkette (1),
- mindestens ein Antriebssystem für den Transport der Strahlungsquellenkette (1) von dem Magazin (10) in ein Führungssystem (12) zur Zuführung der Strahlungsquellenkette (1) bis in die Spitze einer an das Führungssystem (12) angekoppelten Hohlnadel (19),
- und dass eine Vorrichtung (8) zum Durchtrennen der Strahlungsquellenkette (1) an einer definierten Stelle sowie
- eine Kontrolleinrichtung zur Verhinderung einer Beschädigung der Ummantelung der Strahlungsquellen, der Seeds, vorgesehen sind, wobei alle wesentlichen Bestandteile des Gerätes von einem strahlungsabsorbierenden Gehäuse umschlossen sind.

2. Gerät nach Anspruch 1,**dadurch gekennzeichnet, dass** das Antriebssystem für den Transport der Strahlungsquellenkette (1) aus mindestens einer Rolle besteht, welche die Strahlungsquellenkette (1) berührt und durch Reibung schlupffrei von dem Magazin (10) in das Führungssystem (12) befördert oder dass das Antriebssystem für den Transport der Strahlungsquellenkette (1) aus mindestens einem Zahnrad besteht, welches die Strahlungsquellenkette (1) berührt und durch Eingreifen der Zähne in die Hülle der Strahlungsquellenkette (1) diese schlupffrei aus dem Magazin (10) in das Führungssystem (12) befördert.

3. Gerät nach Anspruch 1, **dadurch gekennzeichnet, dass** die Vorrichtung (8) zum Durchtrennen der Strahlungsquellenkette (1) eine voreingestellte Länge als einen Teil der abgewickelten Strahlungsquellenkette (1), der Teilkette, an einer definierten Stelle durchtrennt, wobei die Länge der Teilkette immer ein Vielfaches des Abstandes von Seed zu Seed beträgt.

4. Gerät nach Anspruch 1, **dadurch gekennzeichnet, dass** die Kontrolleinrichtung aus einem Fenster besteht, durch das der Anwender die Lage der Seeds und Abstandshalter erkennen und damit kontrollieren kann und/oder dass die Kontrolleinrichtung opto-elektronische Sensoren zur Feststellung der Position der Seeds und/oder der Abstandshalter in der Strahlungsquellenkette (1) und des Durchtrennungsortes zwischen den einzelnen Seeds der Strahlungsquellenkette (1) ohne Beschädigung der Ummantelung der Seeds aufweist und/oder dass die Kontrolleinrichtung Strahlungsdetektoren zur Feststellung der Position der Seeds in der Strahlungsquellenkette (1) aufweist und/oder dass die Kontrolleinrichtung Mittel zur Blockade des Schneidvorgangs aufweist, die erst dann den Schneidvorgang freigeben, wenn die korrekte Positionierung der Strahlungsquellenkette (1) unter der Schneidvorrichtung festgestellt wurde.

5. Gerät nach Anspruch 1, **dadurch gekennzeichnet, dass** die Funktion der Kontrolleinrichtung unabhängig von der Funktion des Antriebssystems ist.

6. Gerät nach Anspruch 1, **dadurch gekennzeichnet, dass** ein zusätzliches antreibbares Vorschub- bzw. Führungselement (20) in Verbindung mit einem weiteren Führungselement (21) vorgesehen ist, welches in das Führungssystem (12) je nach Anwendungsfall vor oder hinter der Durchtrennvorrichtung über eine Weiche mündet.

7. Gerät nach Anspruch 1, **dadurch gekennzeichnet, dass** ein weiteres Magazin (11) mit einem darauf aufgewickelten antreibbaren Vorschub- bzw. Führungselement (20) vorgesehen ist.

8. Gerät nach Anspruch 1, **dadurch gekennzeichnet, dass** das Magazin (10) für die Strahlungsquellenkette (1) durch einen Zylinder mit einer Nut (22) gebildet ist, deren Tiefe dem Außendurchmesser der Strahlungsquellenkette (1) angepasst ist, wobei die Nut (22) eine Spiralform auf der Zylinderoberfläche beschreibt.

9. Gerät nach den Ansprüchen 1 bis 8, **dadurch gekennzeichnet, dass** neben dem Magazinantrieb (13) mindestens ein weiterer Antrieb (14) für das Weiterbewegen der Strahlungsquellenkette (1) vorgesehen ist.

10. Gerät nach den Ansprüchen 1 bis 9, **dadurch gekennzeichnet, dass** die Durchtrennvorrichtung (8) aus einer exzentrische Kreisbewegungen ausführenden Metallscheibe (28) derart gebildet ist, dass die bioresorbierbaren Abstandshalter (3) der Strahlungsquellenkette (1) in der Mitte sicher durchtrennt, die Metallummantelung der Seeds (2) jedoch nicht beschädigt werden oder dass die Durchtrennvorrichtung (8) aus einem messerartigen Element mit vertikaler Schneidbewegung gebildet ist.

11. Gerät nach den Ansprüchen 1 bis 10, **dadurch gekennzeichnet, dass** die Durchtrennvorrichtung (8) zum thermischen Durchtrennen ausgebildet ist.

12. Gerät nach den Ansprüchen 1 bis 11, **dadurch gekennzeichnet, dass** das Führungssystem (12) auf der der Nadel zugewandten Seite der Führung ein Adapterstück (17) zur Ankopplung einer Implantationshohlnadel (19) aufweist, wobei der Ausgang des Führungssystems (12) über das Adapterstück (17) so gesichert ist, dass nur bei einer angeschlossenen Hohlnadel (19), das Innere der Führungswege geöffnet ist und die Bestandteile der Strahlungsquellenkette (1) dann in den Nadelkanal gelangen können.

13. Gerät nach den Ansprüchen 1 bis 12, **dadurch gekennzeichnet, dass** ein modularer Aufbau derart vorgesehen ist, dass Bauteile, die bei Einsatz direkt am Patienten biologisch kontaminationsgefährdet sind, leicht entfernbar sind und/oder gereinigt/sterilisiert werden können, wie den zur Hohlnadel gerichtet gelegenen Teil des Führungssystems und das Vorschub- bzw. Führungselement zum Einbringen der Teilkette in die Hohlnadel.

14. Gerät nach Anspruch 13, **dadurch gekennzeichnet, dass** vor der Weiche ein Durchtrennsystem vorgesehen ist und über einen weiteren zwischen dem Durchtrennsystem und der Weiche befindlichen Antrieb das abgetrennte Teilstück der Strahlungsquellenkette über die Weiche hinweg zur Nadel transportiert wird und/oder dass hinter der Weiche des Führungssystems zur Nadel hin gerichtet eine Durchtrenneinrichtung vorgesehen ist, wobei das weitere Vorschieben der Teilkette in die Hohlnadel ab hier von dem zusätzlichen Vorschub- bzw. Führungselement (20) geleistet wird.

15. Gerät nach Anspruch 13, **dadurch gekennzeichnet, dass** die Durchtrenneinrichtung nach Anspruch 10 ebenfalls das Vorschub- bzw. Führungselement durchtrennt und so den möglicherweise biologisch kontaminierten Teil dieses Elements entfernbar macht.

16. Nicht therapeutisches Verfahren zum Konfektionieren einer Strahlungsquellenteilkette und zum Einbringen dieser Strahlungsquellenteilkette in Hohlnadeln, wobei die Strahlungsquellen, die Seeds, in definierter Anzahl in einer Strahlungsquellenkette mit Abstandshaltern eingebracht sind, **dadurch gekennzeichnet, dass** eine Vorrichtung (8,9) zur Zuführung und zur Durchtrennung von Strahlungsquellenketten (1) aus einem Magazin (10) an die Hohlnadel (19) angekoppelt wird, wobei die Strahlungsquellenkette entsprechend der gewünschten Anzahl von Seeds aus dem Magazin (10) herausgefördert, der Durchtrennvorrichtung (8) zugeführt, dort abgetrennt und als definierte Teilkette bis in die Hohlnadel eingebracht wird.

17. Verfahren nach Anspruch 16, **dadurch gekennzeichnet, dass** die Bewegung der Strahlungsquellenkette (1)derart gesteuert wird, dass die Strahlungsquellenkette (1) an der vorher definierten Stelle relativ zur Durchtrennvorrichtung positioniert wird und/oder dass der Vorschub der Strahlungsquellenkette (1) in einer Abfolge diskreter Schritte bis zur vorher definierten Trennstelle vorgenommen wird.

18. Verfahren nach Anspruch 16, **dadurch gekennzeichnet, dass** Bauelemente wie Sensor und/oder Detektor (29) zur Überwachung und Einstellung der Durchtrennstelle eingesetzt werden, die den Durchtrennvorgang nur freigeben, wenn der vorherbestimmte Durchtrennort erreicht ist und sich keine Seeds (2) im Trennbereich befinden.

19. Verfahren nach Anspruch 16, **dadurch gekennzeichnet, dass** die Durchtrennvorrichtung solange blockiert bleibt, bis der Durchtrennvorgang freigegeben wurde.

20. Verfahren nach den Ansprüchen 16 bis 19, **dadurch gekennzeichnet, dass** ein Vorschub- bzw. Führungselement (20) aus einem Magazin (11) über mindestens einen Antrieb (15) so gesteuert wird, dass das Element (20) nur hinter der konfektionierten Teilkette (2) in eine Zuführung (16) zur Hohlnadel (19) gelangt.

21. Verfahren nach den Ansprüchen 16 bis 20, **dadurch gekennzeichnet, dass** die Konfektionslänge, bestimmt durch die enthaltene Anzahl von Seeds, vorgewählt und daraus die Vorschubtiefe des Vorschub-/Führungselementes beim Einbringen der Teilkette in die Hohlnadel bestimmt wird.

22. Strahlungsquellenkette (1) zum Einbringen in Hohlnadeln, wobei die Strahlungsquellenkette (1) modular aus wenigstens einer Strahlungsquelle (2) und wenigstens einem bioresorbierbaren Abstandshalter (3) aufgebaut ist, **dadurch**
**gekennzeichnet, dass**
Strahlungsquelle (2) und Abstandshalter (3) der Strahlungsquellenkette (1) von einer kontinuierlichen, bioresorbierbaren Umhüllung (4) umgeben und zusammengehalten sind,
der Abstandshalter (3) langgestreckt mit einer Verjüngung ausgebildet ist, wobei der Durchmesser besagter Verjüngung geringer ist als der Durchmesser des Abstandshalters (3) an seinen Enden,
die Umhüllung der Kette (1) an den Positionen von Strahlungsquellen (2) und Abstandshaltern (3) jeweils verschieden eingefärbt oder markiert ist, so dass die Position der Abstandshalter (3) innerhalb der Kette (1) eindeutig optisch zu identifizieren ist,
die Stirnenden des Abstandshalters (3) in unmittelbarem Kontakt mit den Stirnenden der Strahlungsquelle (2) stehen, wobei die Enden der Abstandshalter (3) und der. Strahlungsquellen (2) eine Form aufweisen, die eine kugelgelenkartige Drehung zulässt und
die Strahlungsquellenkette (1) aufrollbar ausgebildet ist.

23. Strahlungsquellenkette nach Anspruch 22, **dadurch gekennzeichnet, dass** der Abstandshalter (3) einen kreisförmigen Querschnitt aufweist und einen Durchmesser aufweist, der höchstens dem der Strahlungsquelle (2) entspricht und/oder der Abstandshalter (3) von den Enden zur Mitte hin konisch ausgeformt und/oder rohrförmig ist und/oder einen Hohlraum aufweist.

24. Strahlungsquellenkette nach mindestens einem der Ansprüche 22 bis 23, **dadurch gekennzeichnet, dass** die Stirnenden des Abstandshalters (3) eine zu den Stirnenden der Strahlungsquelle (2) konträre Form aufweisen und/oder die Stirnenden des Abstandshalters (3) eine konvexe Form und die Stirnenden der Strahlungsquelle (2) eine konkave Form haben.

25. Strahlungsquellenkette nach mindestens einem der Ansprüche 22 bis 24, **dadurch gekennzeichnet, dass** die Strahlungsquellenkette (1) ein Mittel zur optischen Unterscheidung der Positionen von Abstandshaltern (3) und Strahlungsquellen (2) in der Kette aufweist, das es erlaubt, die Position des Abstandshalters (3) innerhalb der Kette (1) eindeutig optisch zu identifizieren.

26. Strahlungsquellenkette nach Anspruch 25, **dadurch gekennzeichnet, dass** das Mittel zur optischen Unterscheidung der Positionen von Abstandshaltern (3) und Strahlungsquellen (2) eine kontrastierende Form der Abstandshalter (3) und Strahlungsquellen (2) aufweist und/oder eine Markierung oder Einfärbung der Ummantelung aufweist.

27. Strahlungsquellenkette nach einem der Ansprüche 22 bis 26, **dadurch gekennzeichnet, dass** der Abstandshalter (3) ein Region aufweist, die von einer Durchtrennvorrichtung durchtrennt werden kann und/oder der Abstandshalter (3) eine Region aufweist, die mit einer Kraft durchtrennt werden kann, die nicht ausreicht, die Strahlungsquelle (2) zu beschädigen.

28. Strahlungsquellenkette nach Anspruch 27, **dadurch gekennzeichnet, dass** die Strahlungsquellenkette (1) ein Mittel zur Unterscheidung aufweist, welches es erlaubt, die Position der durchtrennbaren Region innerhalb der Kette (1) eindeutig optisch zu identifizieren.

29. Abstandshalter zur Verwendung mit einer Strahlungsquelle (2) zur Bildung einer Strahlungsquellenkette (1), **dadurch gekennzeichnet, dass** der Abstandshalter (3) bioresorbierbar und langgestreckt mit einer Verjüngung ausgebildet ist, wobei der Durchmesser besagter Verjüngung geringer ist als der Durchmesser des Abstandshalters (3) an seinen Enden und die Stirnenden des Abstandshalters (3) für einen unmittelbaren Kontakt mit den Stirnenden der Strahlungsquelle (2) ausgebildet sind, wobei die Enden der Abstandshalter (3) eine Form aufweisen, die eine kugelgelenkartige Drehung zulässt.

30. Abstandshalter nach Anspruch 29, **dadurch gekennzeichnet, dass** der Durchmesser besagten Abstandshalters (3) höchstens dem der Strahlungsquelle (2) entspricht und/oder der Abstandshalter(3) einer konische Form von seinen Enden zur Mitte hin aufweist und/oder inflexibel ausgebildet ist und/oder einen Hohlraum aufweist und/oder Enden aufweist, die zu den Strahlungsquellenenden eine konträre Form aufweisen.

## Claims

1. Device for loading of hollow implantation needles with radiation sources from radiation source chains for interstitial brachytherapy of tissue, **characterized in that**
- at least one supported, drivable, cylindrical magazine (10) for winding the radiation source chain (1),
- at least one drive system for transporting the radiation source chain (1) from the magazine (10) into a guide system (12) for feeding the radiation source chain (1) to the tip of a hollow needle (19) coupled to the guide system (12),
- and a device (8) for separating the radiation source chain (1) at a defined location as well as
- a control device for preventing damage to the sheath of the radiation sources, the seeds, are provided, wherein all essential components of the device are enclosed by a radiation-absorbing housing.

2. Device according to claim 1, **characterized in that** the drive system for transporting the radiation source chain (1) comprises at least one roller, which contacts the radiation source chain (1) and transports the same by friction without slippage from the magazine (10) into the guide system (12) or that the drive system for transporting the radiation source chain (1) comprises at least one gear wheel or toothed rod, which contacts the radiation source chain (1) and by engagement of the teeth with the sheath of the radiation source chain (1) transports the radiation source chain (1) without slippage from the magazine (10) into the drive system (12).

3. Device according to claim 1, **characterized in that** the device (8) for separating the radiation source chain (1) cuts a preset length representing a portion of the unwound radiation source chain (1), the partial chain, at a defined location, wherein the length of the partial chain is always equal to a multiple of the seed to seed distance.

4. Device according to claim 1, **characterized in that** the control device comprises a window, through which the user is able to identify and thereby also control the location of the seeds and spacers and/or that the control device comprises opto-electronic sensors for determining the position of the seeds and/or the spacers in the radiation source chain (1) and of the cutting location between the individual seeds of the radiation source chain (1) without damaging the sheath of the seeds and/or that the control device comprises radiation detectors for determining the position of the seeds in the radiation source chain (1) and/or that the control device comprises means for disabling the cutting operation, which enables the cutting operation only after the correct position of the radiation source chain (1) under the cutting device has been determined.

5. Device according to claim 1, **characterized in that** the function of the control device is independent of the function of the drive system.

6. Device according to claim 1, **characterized in that** an additional drivable push or guide element (20), respectively, is provided in conjunction with an additional guide element (21), which depending on the application merges with the guide system (12) before or after the cutting device through a switch.

7. Device according to claim 1, **characterized in that** an additional magazine (11) is provided having a drivable push or guide element (20), respectively, mounted thereon.

8. Device according to claim 1, **characterized in that** the magazine (10) for the radiation source chain (1) is formed by a cylinder with a groove (22), the depth of the groove being adapted to the outer diameter of the radiation source chain (1), wherein the groove (22) forms a spiral disposed on the surface of the cylinder.

9. Device according to the claims 1 to 8, **characterized in that** in addition to the magazine drive (13) at least one additional drive (14) is provided for moving the radiation source chain (1) on.

10. Device according to the claims 1 to 9, **characterized in that** the cutting device (8) is formed of a metal disk (28) that performs an eccentric circular motion, such that the bio-resorbable spacer (3) of the radiation source chain (1) is reliably cut in the center without damaging the metal sheath of the seeds or that the cutting device (8) is formed by a knife-like element with a linear cutting motion.

11. Device according to claims 1 to 10, **characterized in that** the cutting device (8) is adapted to thermal cutting.

12. Device according to claims 1 to 11, **characterized in that** the guide system (12) is provided with an adapter (17) on the side of the guide facing the needle for connecting a hollow implantation needle (19), wherein the exit of the guide system (12) is secured by the adapter (17) in such a way that only after the connection of a hollow needle (19) the interior of the guide passageways is opened and the components of the radiation source chain (1) are able to reach the needle channel.

13. Device according to claims 1 to 12, **characterized in that** a modular construction is provided so that components which are subject to biological contamination when used directly on a patient are easily removable and/or are easily cleanable/sterilizable, as the section of the guide system oriented towards the hollow needle and the additional drivable push or guide element (20), respectively, for the insertion of the partial chain into the hollow needle.

14. Device according to claim 13, **characterized in that** a cutting system is provided before the switch and that the separated partial section of the radiation source chain is transported past the switch to the needle by an additional drive located between the cutting system and the switch and/or that a cutting system that faces the needle is provided behind the switch of the guide system, wherein the further advance of the partial chain into the hollow needle is effected from here on by the additional push and/or guide element.

15. Device according to claim 13, **characterized in that** the cutting device according to claim 10 also cuts the push or guide element, respectively, and thereby makes it possible to remove the section of the push or guide element, respectively, being possibly biologically contaminated.

16. Non-therapeutic method for packing and introducing partial radiation source chains into a hollow needle, wherein a defined quantity of radiation sources, the seeds, is introduced into a radiation source chain with spacers, **characterized in that** a device (8, 9) for feeding and cutting of radiation source chains (1) from a magazine (10) is coupled to the hollow needle (19), wherein the radiation source chain according to the desired number of seeds is withdrawn from the magazine (10) and fed to the cutting device (8), where the radiation source chain is cut and introduced into the hollow needle as a defined partial chain.

17. Method according to claim 16, **characterized in that** the motion of the radiation source chain (1) is controlled so that the radiation source chain (1) is positioned at the previously defined location relative to the cutting device and/or the radiation source chain (1) is advanced in a sequence of discrete steps up to their previously defined cutting location.

18. Method according to claim 16, **characterized in that** components, such as a sensor and/or detector (29), are employed for monitoring and adjusting the cutting location, which only enable the cutting process after the predetermined cutting location has been reached and no seeds (2) are located in the cutting region.

19. Method according to claim 16, **characterized in that** the cutting device is disabled until the cutting process is released.

20. Method according to claims 16 to 19, **characterized in that** a push or guide element (20), respectively, is controlled from a magazine (11) via at least one drive (15) so that the element (20) reaches an inlet (16) of the hollow needle (19) only past the packaged partial chain (2).

21. Method according to claim 16 to 20, **characterized in that** the packaged length, as determined by the number of seeds contained therein, is pre-selected and the depth of the advance of the push/guide element during the insertion of the partial chain into the hollow needle is determined therefrom.

22. Radiation source chain for injection into a hollow needle, wherein the radiation source chain (1) comprises modularly a radiation source (2) and at least one bio-resorbable spacer (3), **characterized in that**
radiation source (2) and spacer (3) of the radiation source chain (1) are held together and surrounded by a continuous bio-resorbable sheath
the spacer (3) is provided elongated having a throat, wherein the diameter of said throat is smaller than the diameter of the spacer (3) at its ends,
the sheath of the chain (1) is differently colored or marked at the respective positions of radiation sources (2) and spacers (3), so that the position of the spacers (3) within the chain (1) are unambiguously optically identifiable,
the end faces of the spacer (3) are in direct contact with the end faces of the radiation source (2), wherein the ends of the spacer (3) and the source (2) are provided with a shape which allows enables rotation similar to a ball joint, and
the radiation source chain (1) is provided rollable.

23. Radiation source chain according to claim 22, **characterized in that** the spacer (3) is provided with a circular cross section and is provided with a diameter, which is equal to at most the diameter of the employed radiation sources (2) and/or the spacer (3) has a conical shape from either of its ends towards its center and/or a tubular shape and/or is hollow.

24. Radiation source chain according to at least one of the claims 22 to 23, **characterized in that** the end faces of the spacer (3) is provided with a shape contrary to the shape of the end faces of the radiation source (2) and/or the ends faces of the spacer (3) have a convex shape, and the ends of the radiation source have a concave shape.

25. Radiation source chain according to at least one of the claims 22 to 24, **characterized in that** the radiation source chain (1) is provided with a means for optical differentiation of the positions of spacers (3) and radiation sources (2) within the chain, which allows an unambiguous optical identification of a position of the spacer (3) within the chain (1).

26. Radiation source according to claim 25, **characterized in that** the means for optical differentiation of the positions of spacers (3) and radiation sources (2) is provided by a contrasting form of the spacer (3) and the radiation source (2) and/or by a marking or colouring of the sheath.

27. Radiation source according to one of the claims 22 to 26, **characterized in that** the spacer (3) is provided with an area, which is cut in two by a cutting device, and/or the spacer (3) is provided with an area, which is cut in two by a force, which is not sufficient to damage the radiation source (2).

28. Radiation source according to claim 27, **characterized in that** the radiation source chain (1) is provided with a means for differentiation, which allows the unambiguous optical identification of the position of the cutable area within the chain (1).

29. Spacer for use with a radiation source (2) for forming a radiation source chain (1), **characterized in that** the spacer (3) is bio-resorbable and elongated with a throat, wherein the diameter of said throat is smaller than the diameter of the spacer (3) at its ends, and the end faces of spacer (3) are adapted for direct contact with the end faces of the radiation source (2), wherein the end faces of the spacer (3) are provided with a shape, which allows a rotation similar to a ball-joint.

30. Spacer according to claim 29, **characterized in that** the diameter of said spacer (3) is equal to at most the diameter of the radiation source (2) and/or spacer (3) has a conical shape from either of its ends towards its center and/or is inflexible and/or is hollow and/or is provided with ends, which have a contrary form with respect to the radiation source ends.

## Revendications

1. Appareil pour charger des aiguilles creuses d'implantation avec des sources de radiation issues de chaînes de sources de radiation pour la curiethérapie interstitielle de tissu,
**caractérisé en ce qu'**
il comprend :
- au moins un magasin (10) de forme cylindrique monté et susceptible d'être entraîné pour enrouler la chaîne de sources de radiation (1),
- au moins un système d'entraînement pour le transport de la chaîne de sources de radiation (1) du magasin (10) à un système de guidage (12) destiné à guider la chaîne de sources de radiation (1) jusqu'à la pointe d'une aiguille creuse (19) couplée au système de guidage (12),
- un dispositif (8) pour couper la chaîne de sources de radiation (1) en un endroit déterminé, ainsi que
- une installation de contrôle pour éviter d'endommager l'enveloppe des sources de radiation, ou grains,
- tous les composants essentiels de l'appareil étant compris dans un boîtier absorbant le rayonnement.

2. Appareil selon la revendication 1,
**caractérisé en ce que**
le système d'entraînement pour le transport de la chaîne de sources de radiation (1) se compose d'au moins un galet qui touche la chaîne de sources de radiation (1) et la déplace par frottement sans patinage, du magasin (10) au système de guidage (12), ou
le système d'entraînement pour le transport de la chaîne de sources de radiation (1) se compose d'au moins une roue dentée qui touche la chaîne de sources de radiation (1) et la transporte sans patinage, du magasin (10) au système de guidage (12), par pénétration des dents dans l'enveloppe de la chaîne de sources de radiation (1).

3. Appareil selon la revendication 1,
**caractérisé en ce que**
le dispositif (8) pour couper la chaîne de sources de radiation (1) coupe une longueur pré-réglée en tant que partie de la chaîne de sources de radiation (1) déroulée, ou chaîne partielle, en un endroit déterminé, la longueur de la chaîne partielle correspondant toujours à un multiple de la distance entre deux grains.

4. Appareil selon la revendication 1,
**caractérisé en ce que**
l'installation de contrôle se compose d'une fenêtre permettant l'utilisateur de reconnaître la position des grains et des organes d'écartement pour ainsi assurer le contrôle, et/ou
l'installation de contrôle présente des capteurs optoélectroniques pour déterminer la position des grains et/ou des organes d'écartement dans la chaîne de sources de radiation (1) et la position de l'endroit de coupure entre les différents grains de la chaîne de sources de radiation (1) sans endommager l'enveloppe des grains, et/ou
l'installation de contrôle présente des détecteurs de rayonnement pour déterminer la position des grains dans la chaîne de sources de radiation (1), et/ou
l'installation de contrôle présente des moyens de blocage du processus de coupure qui n'autorisent le processus de coupure que lorsque le positionnement correct de la chaîne de sources de radiation (1) a été constaté sous le dispositif de coupe.

5. Appareil selon la revendication 1,
**caractérisé en ce que**
le fonctionnement de l'installation de contrôle est indépendant du fonctionnement du système d'entraînement.

6. Appareil selon la revendication 1,
**caractérisé en ce qu'**
un élément d'avance et de guidage (20) supplémentaire, entraîné en liaison avec un autre élément de guidage (21), débouche dans le système de guidage (12) suivant l'application, en amont ou en aval du dispositif de coupe par l'intermédiaire d'un aiguillage.

7. Appareil selon la revendication 1,
**caractérisé en ce qu'**
un autre magasin (11) portant un élément d'avance et de guidage (20) est susceptible d'être entraîné en étant enroulé sur celui-ci.

8. Appareil selon la revendication 1,
**caractérisé en ce que**
le magasin (10) de la chaîne de sources de radiation (1) est formé par un cylindre présentant une rainure (22) dont la profondeur est adaptée au diamètre extérieur de la chaîne de sources de radiation (1), cette rainure (22) décrivant un tracé en spirale à la surface du cylindre.

9. Appareil selon les revendications 1 à 8,
**caractérisé en ce qu'**
en sus de l'entraînement (13) du magasin, au moins un autre entraînement (14) permet la poursuite du mouvement de la chaîne de sources de radiation (1).

10. Appareil selon les revendications 1 à 9,
**caractérisé en ce que**
le dispositif de coupe (8) est formé d'un disque métallique (28) effectuant un mouvement circulaire excentré de façon à garantir que les organes d'écartement (3) biorésorbables de la chaîne de sources de radiation (1) soient coupés de manière sûre en leur milieu sans toutefois que l'enveloppe métallique des grains (2) soit endommagée, ou
le dispositif de coupe (8) est formé d'un élément en forme de couteau effectuant un mouvement de coupe vertical.

11. Appareil selon les revendications 1 à 10,
**caractérisé en ce que**
le dispositif de coupe (8) est conçu de façon à effectuer une coupe thermique.

12. Appareil selon les revendications 1 à 11,
**caractérisé en ce que**
le système de guidage (12) présente, sur le côté du guidage tourné vers l'aiguille, un adaptateur (17) destiné au couplage d'une aiguille creuse d'implantation (19), la sortie du système de guidage (12) étant assurée par l'adaptateur (17) pour que l'intérieur de la voie de guidage ne soit ouvert que lorsque l'aiguille creuse (19) est raccordée et que les constituants de la chaîne de sources de radiation (11) puissent alors parvenir dans le canal de l'aiguille.

13. Appareil selon les revendications 1 à 12,
**caractérisé en ce qu'**
une construction modulaire permet que les composants susceptibles d'être contaminés biologiquement lors d'une utilisation directe sur le patient puissent être facilement enlevés et/ou nettoyés/stérilisés, comme par exemple la partie du système de guidage située du côté de l'aiguille creuse et l'élément d'avance et de guidage permettant d'introduire la partie de chaîne dans l'aiguille creuse.

14. Appareil selon la revendication 13,
**caractérisé par**
un système de coupe en amont de l'aiguillage, et la partie séparée de la chaîne de sources de radiation est transportée au-delà de l'aiguillage, jusque dans l'aiguille, par un autre moyen d'entraînement situé entre le système de coupe et l'aiguillage, et/ou, en aval de l'aiguillage du système de guidage, vers l'aiguille, une installation de coupe, la poursuite de l'avance de la partie de chaîne dans l'aiguille creuse à partir de ce point étant effectuée par un élément d'avance et de guidage (20) supplémentaire.

15. Appareil selon la revendication 13,
**caractérisé en ce que**
l'installation de coupe selon la revendication 10 coupe également l'élément d'avance et de guidage de sorte à permettre l'élimination d'une partie éventuellement biologiquement contaminée de cet élément.

16. Procédé non thérapeutique permettant la confection d'une chaîne partielle de sources de radiation et permettant l'introduction de cette chaîne partielle de sources de radiation dans des aiguilles creuses, les sources de radiation, ou grains, étant introduites en nombre défini dans une chaîne de sources de radiation avec des organes d'écartement,
**caractérisé en ce qu'**
on couple à l'aiguille creuse (19) un dispositif (8, 9) d'alimentation et de coupe de chaînes de sources de radiation (1) issues d'un magasin (10), la chaîne de sources de radiation étant prélevée du magasin (10) en fonction du nombre souhaité de grains, transférée au dispositif de coupe (8) où elle est coupée puis introduite jusque dans l'aiguille creuse sous la forme d'une chaîne partielle définie.

17. Procédé selon la revendication 16,
**caractérisé en ce qu'**
on commande le mouvement de la chaîne de sources de radiation (1) de façon à positionner cette chaîne de sources de radiation (1) relativement au dispositif de coupe en l'endroit préalablement défini, et/ou on effectue l'avance de la chaîne de sources de radiation (1) par une succession de pas discrets jusqu'à l'endroit de coupe préalablement défini.

18. Procédé selon la revendication 16,
**caractérisé en ce qu'**
on utilise des composants tels que des capteurs et/ou des détecteurs (29) pour surveiller et régler l'endroit de coupe, ces composants n'autorisant l'opération de coupe que lorsque l'on a atteint l'emplacement de coupe prédéterminé et qu'il n'y a pas de grain (2) dans la zone de coupe.

19. Procédé selon la revendication 16,
**caractérisé en ce que**
le dispositif de coupe reste bloqué jusqu'à ce que le processus de coupe ait été autorisé.

20. Procédé selon les revendications 16 à 19,
**caractérisé en ce qu'**
on commande un élément d'avance et de guidage (20) issu d'un magasin (11) par l'intermédiaire d'au moins un moyen d'entraînement (15) de sorte que l'élément (20) ne parvienne à l'aiguille creuse (19) qu'en aval de la chaîne partielle (2) confectionnée, dans un moyen d'alimentation (16).

21. Procédé selon les revendications 16 à 20,
**caractérisé en ce qu'**
on présélectionne la longueur de confection déterminée par le nombre de grains contenus et, sur cette base, on détermine la profondeur d'avance de l'élément d'avance et de guidage permettant d'introduire la chaîne partielle dans l'aiguille creuse.

22. Chaîne de sources de radiation (1) à introduire dans des aiguilles creuses, la chaîne de sources de radiation (1) étant de construction modulaire composée d'au moins une source de radiation (2) et d'au moins un organe d'écartement (3) biorésorbable,
**caractérisée en ce que**
la source de radiation (2) et l'organe d'écartement (3) de la chaîne de sources de radiation (1) sont entourés d'une enveloppe (4) continue biorésorbable qui les maintient réunis,
l'organe d'écartement (3) présente un amincissement longitudinal, le diamètre de l'amincissement étant plus faible que le diamètre de l'organe d'écartement (3) en ses extrémités,
l'enveloppe de la chaîne (1) est colorée ou marquée chaque fois de manière différente aux positions des sources de radiation (2) et des organes d'écartement (3) pour permettre l'identification optique et non équivoque de la position des organes d'écartement (3) au sein de la chaîne (1),
les extrémités frontales de l'organe d'écartement (3) sont en contact direct avec les extrémités frontales de la source de radiation (2), les extrémités des organes d'écartement (3) et des sources de radiation (2) présentant une forme permettant une rotation à la manière d'une articulation sphérique, et
la chaîne de sources de radiation (1) est conçue pour être enroulable.

23. Chaîne de sources de radiation selon la revendication 22,
**caractérisée en ce que**
l'organe d'écartement (3) présente une section circulaire et un diamètre correspondant au maximum à celui de la source de radiation (2), et/ou l'organe d'écartement (3) a une forme conique depuis ses extrémités vers le milieu et/ou est tubulaire et/ou présente une cavité.

24. Chaîne de sources de radiation selon l'une au moins des revendications 22 à 23,
**caractérisée en ce que**
les extrémités frontales de l'organe d'écartement (3) présentent une forme contraire à celle des extrémités frontales de la source de radiation (2), et/ou les extrémités frontales de l'organe d'écartement (3) ont une forme convexe et les extrémités frontales de la source de radiation (2) une forme concave.

25. Chaîne de sources de radiation selon l'une au moins des revendications 22 à 24,
**caractérisée en ce que**
la chaîne de sources de radiation (1) présente un moyen de différentiation optique des positions des organes d'écartement (3) et des sources de radiation (2) dans la chaîne, celui-ci permettant l'identification optique et non équivoque de la position de l'organe d'écartement (3) au sein de la chaîne (1).

26. Chaîne de sources de radiation selon la revendication 25,
**caractérisée en ce que**
le moyen de différentiation optique des positions des organes d'écartement (3) et des sources de radiation (2) présente une forme contrastant celle des organes d'écartement (3) et des sources de radiation (2) et/ou présente un marquage ou une coloration de l'enveloppe.

27. Chaîne de sources de radiation selon l'une des revendications 22 à 26,
**caractérisée en ce que**
l'organe d'écartement (3) présente une région sécable au moyen d'un dispositif de coupe et/ou l'organe d'écartement (3) présente une région sécable par l'application d'une force dont la magnitude n'est pas suffisante pour endommager la source de radiation (2).

28. Chaîne de sources de radiation selon la revendication 27,
**caractérisée en ce que**
la chaîne de sources de radiation (1) présente un moyen de différentiation permettant l'identification optique et non équivoque de la position de la région sécable au sein de la chaîne (1).

29. Organe d'écartement utilisable avec une source de radiation (2) pour former une chaîne de sources de radiation (1),
**caractérisé en ce que**
l'organe d'écartement (3) est biorésorbable et présente un amincissement longitudinal, le diamètre de l'amincissement étant plus faible que le diamètre de l'organe d'écartement (3) en ses extrémités et les extrémités frontales de l'organe d'écartement (3) étant conçues pour un contact direct avec les extrémités frontales de la source de radiation (2), les extrémités des organes d'écartement (3) présentant une forme permettant une rotation à la manière d'une articulation sphérique.

30. Organe d'écartement selon la revendication 29,
**caractérisé en ce que**
le diamètre de l'organe d'écartement (3) correspond au maximum à celui de la source de radiation (2), et/ou l'organe d'écartement (3) présente une forme conique depuis ses extrémités vers le milieu et/ou est de conception rigide et/ou présente une cavité et/ou présente des extrémités ayant une forme contraire à celle des extrémités de la source de radiation.
